(19) **Europäisches Patentamt / European Patent Office / Office européen des brevets**

(11) **EP 4 719 198 B1**

(12) **EUROPEAN PATENT SPECIFICATION**

(45) Date of publication and mention
of the grant of the patent:
**08.07.2026 Bulletin 2026/28**

(21) Application number: **24728578.6**

(22) Date of filing: **22.05.2024**

(51) International Patent Classification (IPC):
**A61B 6/00** *(2024.01)* **G06T 7/00** *(2017.01)*

(52) Cooperative Patent Classification (CPC):
**A61B 6/504; A61B 6/5217; G06T 7/0012**

(86) International application number:
**PCT/EP2024/064002**

(87) International publication number:
**WO 2024/245834 (05.12.2024 Gazette 2024/49)**

(54) **COLLATERAL VESSEL SELECTION IN RETROGRADE CHRONIC TOTAL OCCLUSION TREATMENT PROCEDURES**

KOLLATERALE GEFÄSSAUSWAHL IN RETROGRADEN BEHANDLUNGSVERFAHREN MIT CHRONISCHER TOTALOKKLUSION

SÉLECTION DE VAISSEAUX COLLATÉRALE DANS DES PROCÉDURES DE TRAITEMENT D'OCCLUSION TOTALE CHRONIQUE RÉTROGRADE

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **02.06.2023 EP 23305874**

(43) Date of publication of application:
**08.04.2026 Bulletin 2026/15**

(73) Proprietor: **Koninklijke Philips N.V.**
**5656 AE Eindhoven (NL)**

(72) Inventors:
• **HENDRIKS, Bernardus Hendrikus Wilhelmus**
**5656 AG Eindhoven (NL)**
• **LAI, Marco**
**5656 AG Eindhoven (NL)**
• **GROEN, Joanneke Gerrigje**
**5656 AG Eindhoven (NL)**
• **SIO, Charles Frederik**
**5656 AG Eindhoven (NL)**
• **LUCASSEN, Gerhardus Wilhelmus**
**5656 AG Eindhoven (NL)**
• **PIZAINE, Guillaume Julien Joseph**
**5656 AG Eindhoven (NL)**
• **SPLIETHOFF, Jarich Willem**
**5656 AG Eindhoven (NL)**
• **LARUE, Ruben**
**5656 AG Eindhoven (NL)**

(74) Representative: **Philips Intellectual Property & Standards**
**High Tech Campus 34**
**5656 AE Eindhoven (NL)**

(56) References cited:
**US-A1- 2021 133 961**

• HUANG CHING-CHANG ET AL: "Collateral Channel Size and Tortuosity Predict Retrograde Percutaneous Coronary Intervention Success for Chronic Total Occlusion", vol. 11, no. 1, 1 January 2018 (2018-01-01), US, XP093092838, ISSN: 1941-7640, Retrieved from the Internet <URL:http://dx.doi.org/10.1161/ CIRCINTERVENTIONS.117.005124> DOI: 10.1161/ CIRCINTERVENTIONS.117.005124
• MEGALY MICHAEL ET AL: "Retrograde Approach to Chronic Total Occlusion Percutaneous Coronary Intervention", vol. 13, no. 5, 1 May 2020 (2020-05-01), US, XP093092981, ISSN: 1941-7640, Retrieved from the Internet <URL:http://dx.doi.org/10.1161/ CIRCINTERVENTIONS.119.008900> DOI: 10.1161/ CIRCINTERVENTIONS.119.008900

**Description**

TECHNICAL FIELD

The present disclosure relates generally to performing retrograde chronic total occlusion, CTO, treatment procedures. More specifically, it relates to selecting a collateral vessel for performing a retrograde CTO treatment procedure. A system, a computer-implemented method, and a computer program product, are disclosed.

BACKGROUND

Over time, blood vessels may become obstructed, or in other words, occluded. Blood vessels may become occluded due to a build-up of plaque, or due to a clot in the vessel, for example. Occlusions can occur in blood vessels in various parts of the anatomy, including in the heart, in the brain, and also in peripheral regions such as the leg.

In some cases, an occlusion is partial, and blood flow in the vessel is restricted, but not blocked. For instance, in the heart, a partial occlusion of a coronary artery may result in the heart having to work harder in order to maintain blood flow. However, in more severe cases, blood flow in a vessel may be completely blocked by an occlusion. A chronic total occlusion, or "CTO" is defined as a complete blockage of a blood vessel for a duration of greater than or equal to 3 months.

If a vessel becomes occluded, a treatment procedure may be required. An example of such a treatment procedure is a balloon angioplasty procedure. A balloon angioplasty procedure may be followed by the placement of a stent. A balloon angioplasty procedure has the effect of opening-up a vessel lumen, and the stent maintains the lumen in the opened-up state. This has the effect of restoring blood flow through the vessel. Various interventional devices may be used in such treatment procedures, including a guidewire, a (balloon) catheter, and so forth.

An initial step in such treatment procedures is to pass an interventional device, e.g. a guidewire, through the occlusion. This is referred to as "crossing the occlusion". This step is conventionally performed by crossing the occlusion in an antegrade direction, i.e. by crossing the occlusion in the direction of conventional blood flow. If an antegrade crossing of the occlusion fails, a retrograde crossing of the occlusion, i.e. a crossing of the occlusion against the direction of conventional blood flow, is often attempted as the next step. The retrograde approach is reported as being utilized in 20% to 50% of Peripheral Coronary Interventions "PCI"s that are performed on CTOs worldwide, as mentioned in a document by Megaly, M., et al., "Retrograde Approach to Chronic Total Occlusion Percutaneous Coronary Intervention", Circ. Cardiovasc. Interv. 2020; 13:e008900. As also mentioned in this document, the success rate of a retrograde CTO PCI is 90%, contrasting with a success rate of approximately 70% for the antegrade approach.

However, a retrograde approach to an occlusion has a higher risk of complications as compared to an antegrade approach. Consequently it is important to select the best approach for a given subject. As disclosed e.g. in "Collateral Channel Size and Tortuosity Predict Retrograde Percutaneous Coronary Intervention Success for Chronic Total Occlusion" by Ching-Chang Huang (Circulation: Cardiovascular Interventions 2018, vol. 11, no. 1, 1 January 2018), a physician tries to select the optimal collateral vessel (e.g. a bypass graft, a septal collateral, or an epicardial collateral, in cases in which the occlusion is in a coronary vessel) for approaching the occlusion in a retrograde direction. However, collateral vessels are often difficult to detect in angiographic images because the contrast agent used to generate such images tends not to accumulate close to a CTO. The choice of the optimal collateral vessel is also affected by factors such as the tortuosity of the vessel, the risk of tamponade, the difficulty of wiring in the retrograde direction, the ability to dilate a collateral vessel, and so forth.

Consequently, there is a need for improvements in the selection of collateral vessels for use in retrograde CTO treatment procedures.

SUMMARY OF THE INVENTION

The presently claimed invention is set out in independent claim 1. Preferred embodiments of the invention are set out in the dependent claims. Any subject matter contained in the disclosure that does not fall within the scope of the appended claims is considered as being useful for understanding the invention.

SUMMARY OF THE DISCLOSURE

According to a first aspect of the present disclosure, a system for selecting a collateral vessel for performing a retrograde chronic total occlusion, CTO, treatment procedure, is provided. The system include one or more processors configured to:

receive angiographic image data representing an occlusion in a target vessel;
analyze the angiographic image data to identify one or more candidate collateral vessels for approaching the occlusion in the target vessel in a retrograde direction; and
assign a suitability score to each candidate collateral vessel, the suitability score representing a suitability of the vessel for performing the retrograde CTO treatment procedure on the occlusion.

In the system, the identification of candidate collateral vessels, and the assigning of suitability scores to the candidate collateral vessel, facilitates a physician to select an optimal vessel for performing the procedure. This helps the physician to overcome the challenge of mentally weighing-up the numerous factors that affect its choice.
In a related aspect, the one or more processors are also configured to:

receive second angiographic image data representing an interventional device in the selected collateral vessel;
analyses the second angiographic image data to provide an estimate for an obstruction time period, the obstruction time period representing a total time during which the interventional device has obstructed a lumen of the selected collateral vessel; and
output a warning in response to the estimated obstruction time period exceeding a threshold time period; or
output an indication of a remaining amount of time available to perform the procedure, the remaining amount of time being calculated based on a difference between the threshold time period and the obstruction time period.

As mentioned above, a retrograde approach to an occlusion has a higher risk of complications as compared to an antegrade approach. These complications include, e.g. the risk of myocardial infarction, and the risk of thrombosis of the collateral vessel. Both of these risks increase as a consequence of the collateral vessel being obstructed for a period of time that is too long. In this aspect, the system analyses the second angiographic image data to estimate the total time during which the interventional device has obstructed a lumen of the selected collateral vessel. A warning is outputted if the estimated total time exceeds a threshold time period. Alternatively, an indication of a remaining amount of time available to perform the procedure, is outputted. The warning, and similarly the indication of the remaining amount of time, alerts a physician to the elevated risk of complications during the retrograde CTO treatment procedure.
In accordance with a second aspect of the present disclosure, a system for determining a threshold time period, is provided. The threshold time period represents a maximum total time during which an interventional device can safely obstruct a lumen of a collateral vessel in a retrograde chronic total occlusion, CTO, treatment procedure. In this aspect, the system includes one or more processors configured to:

receive angiographic image data, the angiographic image data representing an occlusion in a target vessel and one or more collateral vessels for approaching the occlusion in the target vessel in a retrograde direction;
input the angiographic image data into a neural network trained to predict the threshold time period for each of the one or more collateral vessels; and
output the threshold time period for each of the one or more collateral vessels in response to the inputting; and
wherein the neural network is trained to predict the threshold time period for each of the one or more collateral vessels based on the inputted angiographic image data.

In accordance with a third aspect of the present disclosure, an alternative system for determining a threshold time period, is provided. The threshold time period represents a maximum total time during which an interventional device can safely obstruct a lumen of a collateral vessel in a retrograde chronic total occlusion, CTO, treatment procedure. In this aspect, the system includes one or more processors configured to:

receive angiographic image data, the angiographic image data representing an occlusion in a target vessel and one or more collateral vessels for approaching the occlusion in the target vessel in a retrograde direction;
extract, from the angiographic image data, one or more vessel parameters for each of the one or more collateral vessels;
input the extracted one or more vessel parameters into a neural network trained to predict the threshold time period for each of the one or more collateral vessels; and
output the threshold time period for each of the one or more collateral vessels in response to the inputting; and
wherein the neural network is trained to predict the threshold time period for each of the one or more collateral vessels based on the inputted one or more vessel parameters.

The systems in these two latter aspects provide a threshold time period for each of the one or more collateral vessels. The threshold time period represents a maximum total time during which an interventional device can safely obstruct a lumen of a collateral vessel in a retrograde chronic total occlusion, CTO, treatment procedure. This information is useful to a physician in weighing-up the choice of collateral vessels for performing a retrograde CTO procedure. For instance, if the system indicates that a particular collateral vessel has a relatively shorter threshold time period as compared to other collateral vessels, the physician may decide to choose a different vessel that has a relatively longer threshold time period in order to safely complete the procedure within its expected duration. If none of the collateral vessels have a threshold time period that is long enough to accommodate the expected duration of the procedure, the physician may even decide to pursue an antegrade approach to the CTO.

Further aspects, features, and advantages of the present disclosure will become apparent from the following description of examples, which is made with reference to the accompanying drawings.

BRIEF DESCRIPTION OF THE DRAWINGS

Fig. 1 is an illustration of a heart, including an example of an occlusion 130 in a target vessel 140, and a path $P_a$ for approaching the occlusion 130 in an antegrade direction, in accordance with some aspects of the present disclosure.

Fig. 2 is an illustration of a heart, including an example of an occlusion 130 in a target vessel 140, and a path $P_r$ for approaching the occlusion 130 in a retrograde direction via a collateral vessel $150_1$, in accordance with some aspects of the present disclosure.

Fig. 3 is a schematic diagram illustrating an example of a system 100 for selecting a collateral vessel for performing a retrograde CTO treatment procedure, in accordance with some aspects of the present disclosure.

Fig. 4 is a flowchart illustrating an example of a computer-implemented method of selecting a collateral vessel for performing a retrograde CTO treatment procedure, in accordance with some aspects of the present disclosure.

Fig. 5 is a schematic diagram illustrating an example of a neural network 170 that is trained to identify the one or more candidate collateral vessels $150_1$, $150_2$, for approaching an occlusion in a target vessel in a retrograde direction, and to assign a suitability score to each candidate collateral vessel, in accordance with some aspects of the present disclosure.

Fig. 6 is a schematic diagram illustrating an example of a system 300 for determining a threshold time period $T_{ObsMax}$, in accordance with some aspects of the present disclosure.

Fig. 7a is a flowchart illustrating a first example of a computer-implemented method of determining a threshold time period $T_{ObsMax}$, in accordance with some aspects of the present disclosure.

Fig. 7b is a flowchart illustrating a second example of a computer-implemented method of determining a threshold time period $T_{ObsMax}$, in accordance with some aspects of the present disclosure.

Fig. 8a is a schematic diagram illustrating an example of a neural network 210 that is trained to predict a threshold time period, $T_{ObsMax}$, for a selected collateral vessel, based on extracted vessel parameters 220, in accordance with some aspects of the present disclosure.

Fig. 8b is a schematic diagram illustrating an example of the analysis of second angiographic image data 390 in order to provide an estimate for an obstruction time period $T_{Obs}$, and the generation of a warning in response to the obstruction time period $T_{Obs}$ exceeding a threshold time period $T_{ObsMax}$, in accordance with some aspects of the present disclosure.

DETAILED DESCRIPTION

Examples of the present disclosure are provided with reference to the following description and Figures. In this description, for the purposes of explanation, numerous specific details of certain examples are set forth. Reference in the specification to "an example", "an implementation" or similar language means that a feature, structure, or characteristic described in connection with the example is included in at least that one example. It is also to be appreciated that features described in relation to one example may also be used in another example, and that all features are not necessarily duplicated in each example for the sake of brevity. For instance, features described in relation to a system, may be implemented in a computer implemented method, and in computer program product, in a corresponding manner.

In the following description, reference is made to various systems that may be used to support a physician in the selection of a collateral vessel for performing a retrograde CTO treatment procedure. In some examples, reference is made to retrograde CTO treatment procedures that are performed on an occlusion that is located in a coronary artery. However, it is to be noted that the systems disclosed herein may be used to support physicians in the selection of collateral vessels for performing retrograde CTO treatment procedures in which occlusions are located in other parts of the anatomy. For instance, the systems disclosed herein may be used to support physicians in the selection of collateral vessels for performing retrograde CTO treatment procedures in which occlusions are located in the leg, or in the brain. In the brain, for example, the systems disclosed herein may be used to support physicians in the selection of collateral vessels within the brain's "collateral circulation", a vascular network which maintains blood flow in the event of a partial blockage of a primary vascular pathway. Moreover, it is to be appreciated that the systems disclosed herein may be used to support physicians in the selection of collateral vessels for performing retrograde CTO treatment procedures in which occlusions are located in other types of blood vessels than an artery. For instance, the systems disclosed herein may be used to support physicians in the selection of collateral vessels for performing retrograde CTO treatment procedures in which occlusions are located in a vein. Thus, the systems disclosed herein may be used to support physicians in the selection of collateral vessels for performing retrograde CTO treatment procedures in which occlusions are located in blood vessels in the body in general.

It is noted that the computer-implemented methods disclosed herein may be provided as a non-transitory computer-readable storage medium including computer-readable instructions stored thereon, which, when executed by at least one processor, cause the at least one processor to perform the method. In other words, the computer-implemented methods may be implemented in a computer program product. The computer program product can be provided by dedicated hardware, or hardware capable of running the software in association with appropriate software. When provided by a processor, the functions of the method features can be provided by a single dedicated processor, or by a single shared processor, or by a plurality of individual processors, some of which can be shared. The functions of one or more of the method features may for instance be provided by processors that are shared within a networked processing architecture such as a client/server architecture, a peer-to-peer architecture, the Internet, or the Cloud.

The explicit use of the terms "processor" or "controller" should not be interpreted as exclusively referring to hardware capable of running software, and can implicitly include, but is not limited to, digital signal processor "DSP" hardware, read only memory "ROM" for storing software, random access memory "RAM", a non-volatile storage device, and the like. Furthermore, examples of the present disclosure can take the form of a computer program product accessible from a computer-usable storage medium, or a computer-readable storage medium, the computer program product providing program code for use by or in connection with a computer or any instruction execution system. For the purposes of this description, a computer-usable storage medium or a computer readable storage medium can be any apparatus that can comprise, store, communicate, propagate, or transport a program for use by or in connection with an instruction execution system, apparatus, or device. The medium can be an electronic, magnetic, optical, electromagnetic, infrared, or a semiconductor system or device or propagation medium. Examples of computer-readable media include semiconductor or solid state memories, magnetic tape, removable computer disks, random access memory "RAM", read-only memory "ROM", rigid magnetic disks and optical disks. Current examples of optical disks include compact disk-read only memory "CD-ROM", compact disk-read/write "CD-R/W", Blu-Ray™ and DVD.

It is also noted that some operations that are performed by the one or more processors of the system disclosed herein may be implemented using artificial intelligence techniques. Suitable techniques may include machine learning techniques, as well as deep learning techniques such as neural networks. For instance, one or more neural networks, may be trained in a supervised, or in some cases unsupervised, manner, to implement the operations that are performed by the one or more processors.

As mentioned above, there is a need for improvements in the selection of collateral vessels for use in retrograde CTO treatment procedures.

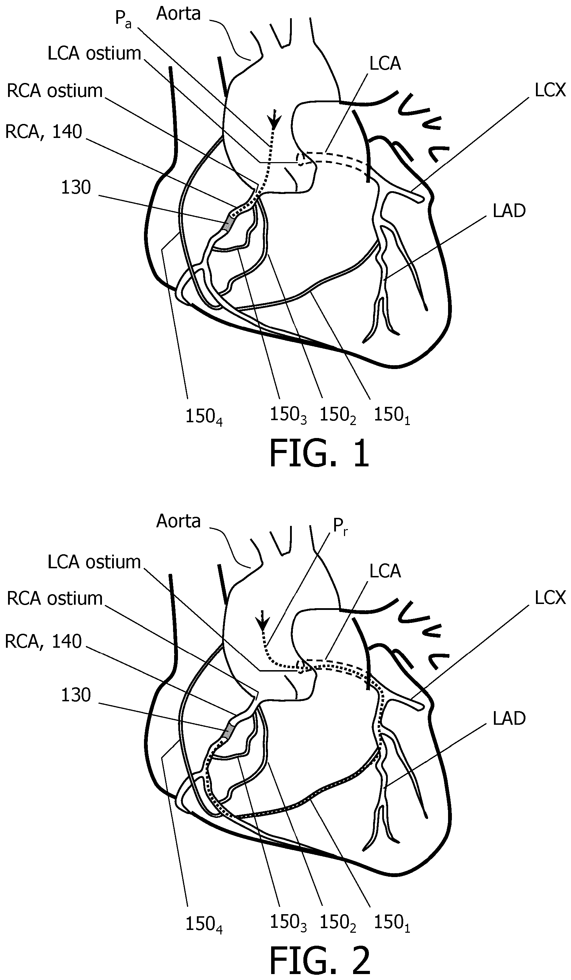

In order to illustrate the challenge of selecting such a vessel, reference is made to Fig. 1, which is an illustration of a heart, including an example of an occlusion 130 in a target vessel 140, and a path $P_a$ for approaching the occlusion 130 in an antegrade direction, in accordance with some aspects of the present disclosure. Various arteries that supply blood to the heart are also illustrated in Fig. 1, including the left coronary artery, LCA, and the right coronary artery, RCA. The LCA extends from its ostium until it branches into the left circumflex artery, LCX, and the left anterior descending artery LAD. In the example illustrated in Fig. 1, an occlusion 130 is located in the RCA.

As described above, an initial step in treating an occlusion such as the occlusion 130 illustrated in Fig. 1, is to pass an interventional device, e.g. a guidewire, through the occlusion. This is referred to as "crossing the occlusion", and is conventionally performed by crossing the occlusion in an antegrade direction, i.e. by crossing the occlusion in the direction of conventional blood flow. This is illustrated in Fig. 1 by the path $P_a$ and its associated arrow. However, if the antegrade crossing of the occlusion fails, a retrograde crossing of the occlusion, i.e. a crossing of the occlusion against the direction of conventional blood flow, is often attempted as the next step. Fig. 1 also illustrates various collateral vessels $150_{1..4}$ that may be used for this purpose. The collateral vessels interconnect the coronary arteries and provide an alternative source of blood supply to the myocardium in cases of occlusive coronary artery disease. In the example illustrated in Fig. 1, these include the septal collateral vessels $150_1$, $150_2$, the epicardial collateral vessel $150_3$, and the bypass graft vessel $150_4$.

Fig. 2 is an illustration of a heart, including an example of an occlusion 130 in a target vessel 140, and a path $P_r$ for approaching the occlusion 130 in a retrograde direction via a collateral vessel $150_1$, in accordance with some aspects of the present disclosure. The heart illustrated in Fig. 2 corresponds to the heart illustrated in Fig. 1. A description of the common features illustrated in both Fig. 2 and in Fig. 1 is not duplicated here for the sake of brevity. In contrast to Fig. 1, the path $P_r$ illustrated in Fig. 2 approaches the occlusion 130 in the retrograde direction, as indicated by its associated arrow. The path $P_r$ approaches the occlusion 130 via the septal collateral vessel $150_1$.

As may be appreciated from the example illustrated in Fig. 2, whilst the collateral vessels $150_{1..4}$ provide alternative paths to the path $P_a$ illustrated in Fig 1 for approaching the occlusion 130, such paths are often difficult to detect in angiographic images because the contrast agent used to generate such images tends not to accumulate close to a CTO. The collateral vessels also typically have a relative smaller lumen size, relatively greater length, and relatively higher tortuosity as compared to the target vessel 140. Thus, the collateral vessels $150_{1..4}$ are typically considered as a second choice after an antegrade approach to the occlusion has failed. A retrograde approach to an occlusion such as that provided via the collateral vessels $150_{1..4}$, also has a higher risk of complications as compared to an antegrade approach. Consequently it is important to select the best approach for a given subject. Here, a physician tries to select the optimal collateral vessel for approaching the occlusion in a retrograde direction. This choice is affected by factors such as the tortuosity of each collateral vessel, the risk of tamponade, the difficulty of wiring in the retrograde direction, the ability to dilate a collateral vessel, and so forth.

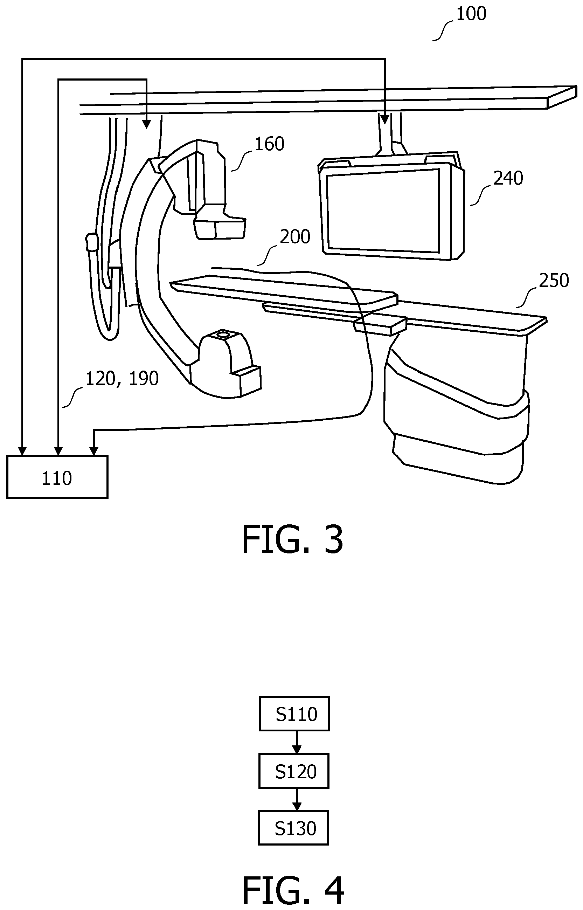

Fig. 3 is a schematic diagram illustrating an example of a system 100 for selecting a collateral vessel for performing a retrograde CTO treatment procedure, in accordance with some aspects of the present disclosure. Fig. 4 is a flowchart illustrating an example of a computer-implemented method of selecting a collateral vessel for performing a retrograde CTO treatment procedure, in accordance with some aspects of the present disclosure. The system 100 illustrated in Fig. 3 includes one or more processors 110. It is noted that operations described as being performed by the one or more processors 110, may also be performed as part of the method illustrated in Fig. 4. Moreover, it is noted that operations described as being performed as part of the method illustrated in Fig. 4, may also be performed by the one or more processors illustrated in Fig. 3.

With reference to Fig. 3, the system 100 for selecting a collateral vessel for performing a retrograde chronic total occlusion, CTO, treatment procedure, comprises one or more processors 110 configured to:

receive S110 angiographic image data 120 representing an occlusion 130 in a target vessel 140;

analyze S120 the angiographic image data 120 to identify one or more candidate collateral vessels $150_{1..i}$ for approaching the occlusion 130 in the target vessel 140 in a retrograde direction; and

assign S130 a suitability score to each candidate collateral vessel $150_{1..i}$, the suitability score representing a suitability of the vessel for performing the retrograde CTO treatment procedure on the occlusion 130.

In the system, the identification of candidate collateral vessels, and the assigning of suitability scores to the candidate collateral vessel, facilitates a physician to select an optimal vessel for performing the procedure. This helps the physician to overcome the challenge of mentally weighing-up the numerous factors that affect its choice.

With reference to the system illustrated in Fig. 3, in the operation S110, the one or more processors 110 receive S110 angiographic image data 120 representing an occlusion 130 in a target vessel 140.

The angiographic image data 120 that is received in the operation S110 may be generated by various types of imaging systems. These include imaging systems that generate angiographic image data that can be reconstructed into a volumetric image, i.e. volumetric image data, and also imaging systems that generate angiographic image data that can be reconstructed into a two-dimensional image, i.e. projection image data. For instance, it may be generated by a magnetic resonance imaging "MRI" system, or by a computed tomography "CT" imaging system, or by a projection X-ray imaging system. In the example illustrated in Fig. 3, the angiographic image data 120 is generated by a projection X-ray imaging system. In examples in which the angiographic image data is generated by a CT, or a projection X-ray, imaging system, the imaging system may be spectral X-ray imaging system. In other words, the angiographic image data 120 may be generated by a spectral CT imaging system, or a spectral X-ray projection imaging system. Spectral X-ray imaging systems generate X-ray attenuation data representing X-ray attenuation within multiple different energy intervals. The X-ray attenuation data from spectral X-ray imaging systems may be processed in order to distinguish between media that have similar X-ray attenuation values when measured within a single energy interval, and which would be indistinguishable in conventional X-ray attenuation data. Thus, the X-ray attenuation data from spectral X-ray imaging systems may be used to provide images with improved specificity to materials such as contrast agent, tissue, bone, and so forth.

In general, the angiographic image data 120 that is received in the operation S110 may represent a single static image, or it may represent a temporal sequence of images, i.e. fluoroscopic images. In some examples, the angiographic image data 120 may be generated instantaneously before being received by the one or more processors 110. For instance, the angiographic image data 120 may represent a live sequence of images. Alternatively, the angiographic image data may have been generated some seconds, minutes, hours, or even days, or a longer period, beforehand. In the latter case, the angiographic image data 120 may have been generated prior to performing a medical procedure on the anatomical region. In this case, the angiographic image data 120 may be referred-to as pre-procedural angiographic image data.

In some examples, the angiographic image data 120 that is received in the operation S110 may be generated subsequent to the injection of a contrast agent into the vasculature of a subject. For instance, angiographic image data that is generated by a CT imaging system, or by a projection X-ray imaging system, may be generated in this manner. The contrast agent highlights blood in the angiographic image data. In some examples, the angiographic image data 120 may represent digital subtraction angiographic "DSA" images. DSA images are generated by subtracting the image intensity values of an X-ray image that has been generated prior to the injection of a contrast agent into the vasculature, from the image intensity values in corresponding positions in X-ray images that are generated after the contrast agent has been injected. Thus, DSA images provide a visualization of blood in the anatomy in the absence of confounding image features such as bone, and which are common to the images before and after contrast agent injection.

The angiographic image data 120 that is received in the operation S110 may be received from various sources, including from a medical imaging system such as the imaging systems described above. Alternatively, the angiographic image data 120 may be received from another source, such as a database, a computer readable storage medium, the internet, the cloud, and so forth. The angiographic image data 120 may be received via any form of data communication, including wired and wireless communication. By way of some examples, when wired communication is used, the communication may take place via an electrical or optical cable, and when wireless communication is used, the communication may for example be via RF or infrared signals.

The angiographic image data 120 that is received in the operation S110 represents an occlusion in a target vessel. The angiographic image data may represent a CTO in the right coronary artery 140, as illustrated in the example in Fig. 1. In other examples, the angiographic image data may represent an occlusion in another vessel in the anatomy.

Returning to the system illustrated in Fig. 3, in the operation S120, the one or more processors 110 analyze the angiographic image data 120 in order to identify one or more candidate collateral vessels $150_{1..i}$ for approaching the occlusion 130 in the target vessel 140 in a retrograde direction.

In one example, the angiographic image data 120 that is received in the operation S110 includes cardiac angiographic image data. In this example, the candidate collateral vessels that are identified in the operation S120 may include: one or more septal collateral vessels $150_1$, $150_2$ and/or one or more epicardial collateral vessels $150_3$ and/or one or more bypass graft vessels $150_4$. Examples of these vessels are illustrated in the example in Fig. 1. In other examples, the angiographic image data 120 that is received in the operation S120 may represent a different region of the anatomy, and thus different collateral vessels may be identified in the operation S120.

In one example, the operation S120 is performed on segmented angiographic image data. In this example, the one or more processors 110 are configured to segment the angiographic image data 120 to identify the target vessel 140, and the occlusion 130, and the one or more candidate collateral vessels $150_{1..i}$. The one or more processors analyze the segmented angiographic image data to identify the one or more candidate collateral vessels $150_{1..i}$. The segmentation that is performed in accordance with this example improves the distinction between different anatomical regions in the anatomical image data. Consequently, performing the operation S120 on segmented angiographic image data, provides a more reliable identification of the collateral vessels $150_{1..i}$. Various segmentation techniques may be used for this purpose, including region growing, thresholding, feature detection, model-based segmentation, and segmentation using a trained neural network such as a U-Net.

In a related example, labels may be applied to the segmented target vessel 140, occlusion 130, and one or more candidate collateral vessels $150_{1..i}$. A graphical representation of the segmented angiographic image data 120 including the corresponding labels is also outputted. This helps to inform a physician of the different options for reaching the occlusion.

In another example, the angiographic image data 120 that is received in the operation S110 is generated by a spectral X-ray imaging system and includes X-ray attenuation data representing X-ray attenuation within multiple different energy intervals. In this example, the one or more processors 110 are configured to analyze the angiographic image data 120 in the operation S120 by applying a material decomposition algorithm to the angiographic image data 120 in order to identify the target vessel 140, and the occlusion 130, and the one or more candidate collateral vessels $150_{1..i}$. Various material decomposition algorithms are known for this purpose.

In one example, the one or more processors 110 are configured to analyze the angiographic image data 120 in order to identify the one or more candidate collateral vessels $150_{1..i}$, by:

identifying one or more vessels having a connection to a distal position with respect to the occlusion 130, and for each identified vessel;
establishing a presence of a path between the connection and a proximal position with respect to the occlusion; and assigning the vessel as a candidate collateral vessel $150_{1..i}$ based on the presence of the path.

These operations may be performed using various image processing techniques. For instance, with reference to the example illustrated in Fig. 3, the above operations may start with identifying the coronary arteries LCA, LCX, LAD, and RCA. This may be performed using the segmentation techniques described above such as a model-based segmentation. Subsequently, the location of the occlusion 130 in the target vessel 140 may be identified by evaluating an intensity gradient along each of the arteries. The location of the occlusion 130 may be identified as the location of an anomalous change in the intensity gradient. Starting at a distal position with respect to the occlusion, one or more vessels having a connection to a distal position with respect to the occlusion 130, may then be identified based on the presence of branches in the target vessel. At each branch, it may be established whether there is a path to a proximal position with respect to the occlusion. This operation may be determined by evaluating a connectivity metric for the path, i.e. a value representing a likelihood that neighboring pixels along the path are connected. If such a path exists, the vessel may be deemed to be a candidate collateral vessel.

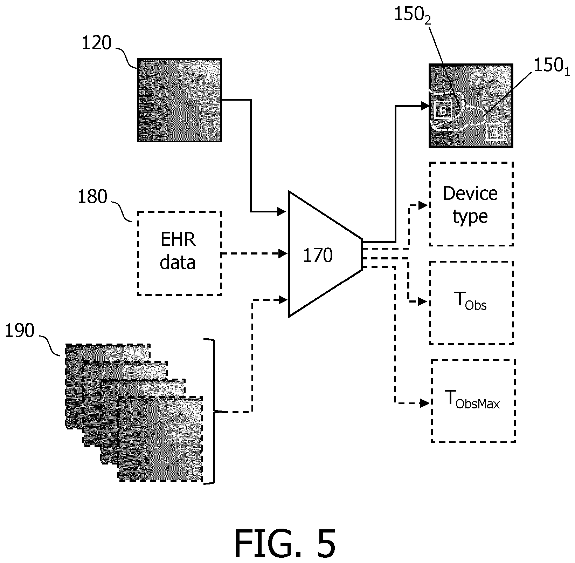

In another example, the operation of analyzing S120 the angiographic image data 120 to identify the one or more candidate collateral vessels $150_{1..i}$ for approaching the occlusion 130 in the target vessel 140 in a retrograde direction, is performed using a neural network 170. This example is described in more detail below with reference to Fig. 5.

Returning to the system illustrated in Fig. 3, in the operation S130, the one or more processors 110 assign a suitability score to each candidate collateral vessel $150_{1..i}$. The suitability score represents a suitability of the vessel for performing the retrograde CTO treatment procedure on the occlusion 130.

The suitability scores for each candidate collateral vessel $150_{1..i}$ may be evaluated based on various factors, including, for instance, one or more of:

a tortuosity of the candidate collateral vessel;
a tamponade risk value for the candidate collateral vessel;
a complexity of routing an interventional device along the candidate collateral vessel;
an ability to dilate the candidate collateral vessel;
electronic health record data relating to the occlusion;
blood flow data for the candidate collateral vessel.

These factors may be evaluated in various ways. For instance, a metric representing a tortuosity of a collateral vessel may be evaluated from the angiographic image data 120 by using image processing techniques to evaluate an integrated amount of deviation along the vessel path from a straight path, or a maximum value of the deviation from a straight path at one or more points along the vessel, or a radius of curvature of the vessel along one or more sections of the vessel, or the presence of a corkscrew-shape along the vessel path.

Tamponade occurs when there is a perforation of a collateral vessel. The risk of tamponade may therefore be evaluated based on factors such as the thickness of the collateral vessel wall (a relatively thin vessel wall being associated with a relatively higher tamponade risk), the collateral vessel tortuosity (a relatively higher amount of tortuosity being associated with a relatively higher tamponade risk), the use of anti-coagulant in the CTO treatment procedure, and the type of the collateral vessel (e.g. bypass graft). The risk of collateral vessel perforation is also affected by the angle between the collateral vessel and the target vessel in which the occlusion is located. Thus, a relatively higher risk of vessel perforation, and consequently tamponade, may be associated with a collateral vessel if the angle between the collateral vessel and the (target) vessel in which the occlusion is located exceeds a specified angle, such as 90 degrees. A tamponade risk value for a collateral vessel may therefore be evaluated from the angiographic image data 120 by using image processing techniques to quantify such factors.

A metric representing a complexity of routing an interventional device along a collateral vessel may be evaluated from the angiographic image data 120 by using image processing techniques to assign complexity metrics to sections of the candidate collateral vessel. For instance, branches along the candidate collateral vessel, including a branch selected by entering the collateral vessel, and a branch selected by leaving the collateral vessel, may be assigned a complexity metric based on a diameter of the collateral vessel, an angle through which the path deviates, a distance from a distal end of the collateral vessel to a distal cap of the occlusion, and so forth.

Some collateral vessels may be capable of being dilated using e.g. a balloon, or via intravenous drug injection, and this facilitates increased maneuverability of an interventional device along the collateral vessel. Vessels that are capable of being dilated may therefore be deemed to be more suitable for performing a retrograde CTO treatment procedure than vessels that do not have this capability. The ability to dilate a collateral vessel may be determined from a database.

Electronic health record "EHR" data relating to the occlusion, may also be used to determine a suitability of a given collateral vessel for performing a retrograde CTO treatment procedure. The EHR data may include elements such as body mass index, smoking history, age, gender, and so forth. The EHR data may also include elements relating to clinical investigations that have been performed on a subject, such as ECG data, data relating to characteristics of the collateral vessels, such as their tortuosity, lumen diameter, the presence of artifacts such as calcifications, the presence of implanted devices such as stents, the outcomes of historic procedures on the subject, such as bleeding incidence, and so forth. The EHR data may include elements relating to whether a retrograde CTO procedure has been attempted before in a given collateral vessel. If a vessel has been used before, this may render the vessel less suitable for use in a retrograde CTO procedure than a vessel that has not been used before due to an elevated risk of rupture of the previously-used vessel. The EHR data may include elements relating to blood flow for a collateral vessel. Blood flow data may be used to determine a suitability of a vessel for performing a retrograde CTO procedure on a collateral vessel. The blood flow data may include a transit time for the vessel, for example. The transit time represents the time taken for an injected contrast agent bolus to travel along the vessel, and provides an indication of the blood flow rate along the vessel. If a given vessel has a relatively long transit time in comparison to other vessels, this may be indicative of the vessel being unsuitable for use in performing a retrograde CTO treatment procedure on the occlusion since it may be indicative of a relatively narrow vessel along which it may be difficult to navigate an interventional device. The blood flow data may be evaluated from the angiographic image data 120 by using image processing techniques to determine the time taken for an injected contrast agent bolus to pass between a proximal position in the vessel and a distal position in the vessel. The EHR data may include elements relating to intravascular imaging procedures that have been performed on the target vessel, or on the collateral vessels. For instance, results obtained from intravascular ultrasound "IVUS" imaging data, or optical coherence tomography "OCT" imaging data may be used. The contributions from the individual elements of EHR data such as those described above may be weighted in order to provide a metric representing a suitability of each collateral vessel, or alternatively the data may be inputted into a neural network, as described in more detail below with reference to Fig. 5. Various elements of EHR data such as those described above may be weighted based on the considerations described above to provide a combined weighted suitability of a given collateral vessel for performing a retrograde CTO treatment procedure.

In another example, the operation of assigning S 130 a suitability score to each candidate collateral vessel $150_{1..i}$, is performed using a neural network 170. This example is described in more detail below with reference to Fig. 5.

Returning to the system illustrated in Fig. 3, after having assigned the suitability score(s) to the candidate collateral vessel(s) in the operation S130, the one or more processors 110 may then output the assigned suitability score(s). The suitability score(s) may be outputted in various ways. In some examples, the suitability scores are outputted graphically. For instance, in one example, the suitability score(s) may be outputted in the form of a ranked list that includes an identification of each collateral vessel, and the corresponding score. In another example, a graphical representation of the collateral vessel(s) may be generated and collateral vessel(s) may be labelled, or color-coded with the corresponding suitability score(s). The graphical representation of the collateral vessel(s) may be generated from the angiographic image data 120. In one example the graphical representation of the collateral vessel(s) includes a synthesized image of the collateral vessel(s) and the suitability score(s) are outputted by color-coding the collateral vessel(s) according to their suitability.

In another example, the one or more processors 110 are configured to output an indication of a recommended orientation of a projection X-ray imaging system 160 for acquiring projection X-ray images of one or more of the identified candidate collateral vessels $150_{1..i}$ during the CTO treatment procedure. Projection X-ray imaging systems are often used in CTO treatment procedures due to their relatively lower X-ray dose as compared to CT imaging systems. However, the absence of depth information in projection X-ray images requires a projection X-ray imaging system to have a suitable orientation with respect to the anatomy in order to provide useful images of the vessels during the procedure. In this example, the outputted recommended orientation helps to reduce the amount of repositioning of the projection X-ray imaging system that is required in order to obtain useful images of the collateral vessel(s) during the procedure.

In this example, the recommended orientation of a projection X-ray imaging system 160 may be determined using various techniques. In one technique, a 3D model representing the heart and the collateral vessel(s) $150_{1..i}$, is used. In this technique, the angiographic image data 120 includes projection image data generated by a projection X-ray imaging system. The projection image data is registered to the 3D model in order to determine a current orientation of the projection X-ray imaging system with respect to the collateral vessel(s) in the 3D model. A database that stores a recommended orientation for acquiring X-ray images of each collateral vessel in the 3D model, is then consulted in order to determine adjustments to the current orientation of the projection X-ray imaging system that are required in order to acquire the projection X-ray images of the collateral vessel(s). The recommended orientations for the collateral vessels in the 3D model may be provided by an expert physician. The required adjustments are then outputted. The required adjustments may be outputted by displaying the required adjustments on a monitor, or by outputting control signals for automatically adjusting the orientation of the projection X-ray imaging system, for example.

In another technique, the angiographic image data 120 includes volumetric image data. The volumetric image data may be generated by a CT imaging system, or an MRI imaging system, for example. In this technique, a recommended orientation for acquiring projection X-ray images of each candidate collateral vessel $150_{1..i}$ may be determined by reconstructing the volumetric image data into a volumetric image, and calculating an orientation of a virtual plane passing through the candidate collateral vessel that minimizes a least squares error difference between each of multiple positions along the vessel, and the plane. The orientation of a vector that is oriented normally with respect to the virtual plane is then calculated. This provides the recommended orientation of the projection X-ray imaging system 160 for acquiring projection X-ray images of the vessel during the CTO treatment procedure.

In another example technique, the angiographic image data 120 is inputted into a neural network that is trained to predict a recommended orientation of a projection X-ray imaging system 160 for acquiring projection X-ray images of each of one or more candidate collateral vessels $150_{1..i}$ in the angiographic image data 120. The training data that is used to train the neural network includes angiographic training images representing one of more collateral vessels for each of a plurality of different subjects, and for each training image, a corresponding recommended orientation. The recommended orientations in the training data that are used in this technique may be provided by an expert.

In another example, the one or more processors 110 are configured to output an indication of a risk score for one or more of the identified candidate collateral vessels $150_{1..i}$. The risk score represents a risk of a medical complication arising from use of the vessel in the retrograde CTO treatment procedure.

In this example, the risk score that is outputted may represent a risk of various medical complications such as myocardial infarction, thrombosis, pericardiocentesis, contrast-induced nephropathy of the candidate collateral vessel(s), and so forth. An indication of a risk score may also be outputted for a risk of a medical complication arising from performing an antegrade CTO treatment procedure on the occlusion. This facilitates a physician to evaluate the comparative risks of performing the CTO treatment procedure using the antegrade and retrograde approaches.

The risk score may be evaluated for the collateral vessel(s), and for the antegrade approach using various techniques. In one technique, the risk score is evaluated using a database of historical CTO procedure outcome data, and corresponding EHR data for historical procedures that have been performed on multiple different subjects. In this technique, the database is consulted using EHR data relating to the occlusion 130 to identify historical CTO procedure outcome data for one or more similar subjects. The risk score is then evaluated based on the outcome data for the similar subjects. The one or more similar subjects may be identified by applying a similarity metric to the EHR data. A similarity metric such as the Dice coefficient, may be used. The similarity metric may be used to identify similar subjects based on elements of the EHR data such as age, gender, weight, height, body mass index, and type of collateral vessel, for example. The risk score may be evaluated by applying a statistical analysis to the outcome data for the similar subjects. If a single subject is identified using this technique, the outcome data for the single subject may be outputted.

In another example technique, the angiographic image data 120 is inputted into a neural network that is trained to predict the risk score for each of one or more vessels in the angiographic image data 120. The training data that is used to train the neural network may include angiographic training images representing one of more vessels for each of a plurality of different subjects, and for each training image, a corresponding risk score. The risk scores in the training data may be provided by experts, or they may be curated from historical outcome data for procedures that have been performed on the vessels.

As mentioned above, in some examples, the operations of analyzing S120 the angiographic image data 120 to identify the one or more candidate collateral vessels $150_{1..i}$, and assigning S 130 a suitability score to each candidate collateral vessel $150_{1..i}$, are performed using a neural network 170. These examples are now described with reference to Fig. 5, which is a schematic diagram illustrating an example of a neural network 170 that is trained to identify the one or more candidate collateral vessels $150_1$, $150_2$, for approaching an occlusion in a target vessel in a retrograde direction, and to assign a suitability score to each candidate collateral vessel, in accordance with some aspects of the present disclosure. With reference to Fig. 5 and Fig. 3, in these examples, the one or more processors 110 are configured to analyze the angiographic image data 120 to identify one or more candidate collateral vessels $150_{1..i}$ and/or to assign a suitability score to each candidate collateral vessel, by:

inputting the angiographic image data 120 into at least one neural network 170; and
outputting the identified one or more candidate collateral vessels $150_{1..i}$ and/or the assigned one or more suitability scores, in response to the inputting; and
wherein the at least one neural network 170 is trained to identify the one or more candidate collateral vessels for approaching the occlusion in the target vessel in a retrograde direction and/or to assign a suitability score to each candidate collateral vessel, based on the inputted angiographic image data.

In the example illustrated in Fig. 5, the neural network 170 may include one or more architectures such as a convolutional neural network "CNN", a recurrent neural network "RNN", a variational autoencoder "VAE", a transformer, and so forth. In the example illustrated in Fig. 5, angiographic image data 120 is inputted into the neural network 120, as illustrated in the top left-hand side of Fig. 5. In the illustrated example, the angiographic image data 120 represents a single static image. The angiographic image data 120 may alternatively represent a temporal sequence of images. As illustrated in the top right-hand side of Fig. 5, in response to the inputting, the neural network 170 outputs one or more identified candidate collateral vessels $150_{1..i}$ and/or one or more suitability scores that have been assigned to the collateral vessels $150_{1..i}$. In the illustrated example, the candidate collateral vessels $150_1$ and $150_2$ have been identified, and these are outputted in the form of dashed lines on a graphical representation of the angiographic image data 120. In the illustrated example, the suitability scores 3, and 6, have been assigned to the candidate collateral vessels $150_1$ and $150_2$, and these have been outputted by labelling the respective vessels in the graphical representation of the angiographic image data 120. In other examples. the neural network 170 may receive further input data, and may output further data. Some examples of this data are illustrated in Fig. 5 via the dashed lines. These examples are described in more detail below.

The training of the at least one neural network 170 is now detailed. In general, the training of a neural network involves inputting a training dataset into the neural network, and iteratively adjusting the neural network's parameters until the trained neural network provides an accurate output. Training is often performed using a Graphics Processing Unit "GPU" or a dedicated neural processor such as a Neural Processing Unit "NPU" or a Tensor Processing Unit "TPU". Training often employs a centralized approach wherein cloud-based or mainframe-based neural processors are used to train a neural network. Following its training with the training dataset, the trained neural network may be deployed to a device for analyzing new input data during inference. The processing requirements during inference are significantly less than those required during training, allowing the neural network to be deployed to a variety of systems such as laptop computers, tablets, mobile phones and so forth. Inference may for example be performed by a Central Processing Unit "CPU", a GPU, an NPU, a TPU, on a server, or in the cloud.

The process of training the neural network(s) 170 described above therefore includes adjusting its parameters. The parameters, or more particularly the weights and biases, control the operation of activation functions in the neural network. In supervised learning, the training process automatically adjusts the weights and the biases, such that when presented with the input data, the neural network accurately provides the corresponding expected output data. In order to do this, the value of the loss functions, or errors, are computed based on a difference between predicted output data and the expected output data. The value of the loss function may be computed using functions such as the negative log-likelihood loss, the mean squared error, or the Huber loss, or the cross entropy loss. During training, the value of the loss function is typically minimized, and training is terminated when the value of the loss function satisfies a stopping criterion. Sometimes, training is terminated when the value of the loss function satisfies one or more of multiple criteria.

Various methods are known for solving the loss minimization problem such as gradient descent, Quasi-Newton methods, and so forth. Various algorithms have been developed to implement these methods and their variants including but not limited to Stochastic Gradient Descent "SGD", batch gradient descent, mini-batch gradient descent, Gauss-Newton, Levenberg Marquardt, Momentum, Adam, Nadam, Adagrad, Adadelta, RMSProp, and Adamax "optimizers" These algorithms compute the derivative of the loss function with respect to the model parameters using the chain rule. This process is called backpropagation since derivatives are computed starting at the last layer or output layer, moving toward the first layer or input layer. These derivatives inform the algorithm how the model parameters must be adjusted in order to minimize the error function. That is, adjustments to model parameters are made starting from the output layer and working backwards in the network until the input layer is reached. In a first training iteration, the initial weights and biases are often randomized. The neural network then predicts the output data, which is likewise, random. Backpropagation is then used to adjust the weights and the biases. The training process is performed iteratively by making adjustments to the weights and biases in each iteration. Training is terminated when the error, or difference between the predicted output data and the expected output data, is within an acceptable range for the training data, or for some validation data. Subsequently the neural network may be deployed, and the trained neural network makes predictions on new input data using the trained values of its parameters. If the training process was successful, the trained neural network accurately predicts the expected output data from the new input data.

In one example, the at least one neural network 170 is trained to identify the one or more candidate collateral vessels and/or to assign a suitability score to each candidate collateral vessel, by:

receiving training data, the training data comprising, for each of a plurality of subjects: angiographic image data representing an occlusion in a target vessel;
receiving ground truth data corresponding to the training data, the ground truth data comprising, for each of the plurality of subjects: an identification of one or more collateral vessels for approaching the occlusion in the target vessel in a retrograde direction and/or a suitability score for each collateral vessel, the suitability score representing a suitability of the vessel for performing a retrograde CTO treatment procedure on the occlusion; and
for each of a plurality of the subjects:

inputting the training data, into the at least one neural network; and
adjusting parameters of the at least one neural network based on a value of a loss function representing a difference between the one or more collateral vessels identified by the at least one neural network and/or the suitability score assigned to each collateral vessel by the at least one neural network, and the corresponding ground truth data; and
repeating the inputting, and the adjusting, until a stopping criterion is met.

The training data that is used in this example may be curated from historical procedures and in which the ground truth candidate collateral vessels and/or suitability scores have been provided by an expert. The ground truth suitability scores may be evaluated based on the factors described above. For instance they may be evaluated based on one or more of:

a tortuosity of the candidate collateral vessel;
a tamponade risk value for the candidate collateral vessel;
a complexity of routing an interventional device along the candidate collateral vessel;
an ability to dilate the candidate collateral vessel;
electronic health record data relating to the occlusion;
blood flow data for the candidate collateral vessel.

Alternatively, the ground truth suitability scores may be assigned based on the experience of the expert. For instance, the expert may provide the suitability scores of the vessels in the form of a ranked list wherein the ranking corresponds to their suitability.

In a related example, the at least one neural network 170 is trained to identify the one or more candidate collateral vessels and/or to assign a suitability score to each candidate collateral vessel, based further on electronic health record data 180 relating to the occlusion 130. In this example, the one or more processors 110 are further configured to:

receive electronic health record data 180 relating to the occlusion 130; and
input the electronic health record data into the at least one neural network 170; such that the outputted one or more candidate collateral vessels and/or the assigned one or more suitability scores, is based further on the electronic health record data.

This example is illustrated in Fig. 5, and wherein EHR data 180 is inputted into the one or more neural networks 170 in the central left-hand portion of Fig. 5. The various types of EHR data described above may be inputted into the one or more neural networks 170 in accordance with this example. In one example the EHR data includes electrocardiogram "ECG" data relating to the occlusion. The ECG data may be inputted in the form of a graphical representation of an ECG signal. The use of the EHR data improves the accuracy of the outputs of the at least one neural network 170 since its outputs may be generated based on trends that identified in the EHR data during training. In this example, the at least one neural network 170 may be trained to identify the one or more candidate collateral vessels and/or to assign a suitability score to each candidate collateral vessel, by including in the training data described above, electronic health record data 180 relating to the occlusion 130.

In another related example, the at least one neural network 170 is trained to predict a recommended type of an interventional device for use in the CTO treatment procedure, based on the inputted angiographic image data 120. In this example, the one or more processors 110 are configured to output the recommended type of the interventional device for use in the CTO treatment procedure, in response to the inputting of the angiographic image data 120 into the at least one neural network 170.

This example is illustrated in Fig. 5, and wherein a Device type is outputted by the one or more neural networks 170, as illustrated in the right-hand portion of Fig. 5. Various types of interventional devices may be outputted in accordance with this example, including for instance a type of (balloon) catheter, a type of guidewire, a type of thrombectomy device, and so forth. The type of the device that is outputted may include a model number, or a specification for a thickness, stiffness, or tip-shape of the interventional device, for example. The outputting of the Device type in accordance with this example provides a physician with additional guidance during the procedure. In this example, the at least one neural network 170 is trained to predict the recommended type of an interventional device for use in the CTO treatment procedure, by including in the training data described above, ground truth data representing a recommended type of the interventional device for use in the CTO treatment procedure, and evaluating the loss function based further on a difference between the recommended type of interventional device predicted by the one or more neural networks, and the recommended type of the interventional device for use in the CTO treatment procedure from the ground truth data.

As mentioned above, a retrograde approach to an occlusion has a higher risk of complications as compared to an antegrade approach. These complications include, e.g. the risk of myocardial infarction, and the risk of thrombosis of the collateral vessel. Both of these risks increase as a consequence of the collateral vessel being obstructed for a period of time that is too long. In another example, the system analyses second angiographic image data to estimate the total time during which the interventional device has obstructed a lumen of the selected collateral vessel, and outputs a warning if this exceeds a threshold time period, or an indication of the remaining amount of time available to perform the procedure. The warning, and similarly the indication of the remaining amount of time available to perform the procedure, alerts a physician to the elevated risk of complications during the retrograde CTO treatment procedure.

In this example, the one or more processors 110 of the system described above with reference to Fig. 3, are configured to:

receive second angiographic image data 190 representing an interventional device 200 in the selected collateral vessel;
analyze the second angiographic image data 190 to provide an estimate for an obstruction time period $T_{obs}$, the obstruction time period representing a total time during which the interventional device 200 has obstructed a lumen of the selected collateral vessel; and
output a warning in response to the estimated obstruction time period exceeding a threshold time period $T_{ObsMax}$; or
output an indication of a remaining amount of time available to perform the procedure, the remaining amount of time being calculated based on a difference between the threshold time period $T_{ObsMax}$ and the obstruction time period $T_{obs}$.

In this example, the warning may be provided in various ways, including visually, such as via a display device such as the monitor 240 illustrated in Fig. 3, or audially, and so forth. The warning, and similarly the indication of the remaining amount of time available to perform the procedure, alerts a physician to the elevated risk of complications during the retrograde CTO treatment procedure.

In this example, the second angiographic image data 190 may represent a temporal sequence of images. The second angiographic image data 190 may be analyzed to provide an estimate for an obstruction time period $T_{obs}$ using various techniques. In one technique, image processing techniques such as feature detection may be used to detect the position of the interventional device in the temporal sequence of images in the second angiographic image data 190. In this technique, the obstruction time period $T_{obs}$ may be estimated by detecting the position of the interventional device in relation to the selected collateral vessel in the images in the temporal sequence, and accumulating the total time during which the interventional device 200 is within the selected collateral vessel. The detection of the position of the interventional device may be facilitated by providing one or more radiopaque markers on the interventional device. In another technique described in more detail below, the obstruction time period $T_{obs}$ may be estimated using a neural network.

In this example, the threshold time period $T_{ObsMax}$ represents a maximum total time during which an interventional device can safely obstruct a lumen of a collateral vessel in a retrograde chronic total occlusion, CTO, treatment procedure. In this example, the term "obstructs" is to be construed as encompassing a range of obstructive situations, including "at least partially blocking", and "completely blocking". In this example, the term "safely obstruct" is to be construed as encompassing a safety margin that provides e.g. sufficient time for a physician to remove the interventional device from the lumen before the onset of the medical complication. For instance, it may represent 90% of the estimated time before myocardial infarction is expected to occur.

The value of the threshold time period $T_{ObsMax}$ may be estimated using various techniques. In one example that is described below, the value of the threshold time period $T_{ObsMax}$ is estimated based on the ratio of the cross-sectional area of the interventional device used in the CTO treatment procedure to the cross sectional area of the collateral vessel. As reported in a document by Wustmann, K., et al., "Is There Functional Collateral Flow During Vascular Occlusion in Angiographically Normal Coronary Arteries?", Circulation, 2003, 107, pages 2213-2220, in one study, the coronary artery of 100 individuals was temporarily occluded with an angioplasty balloon for 1 minute. The flow provided by collateral circulation during the temporary occlusion was approximately 18% of the baseline flow, as measured by the collateral flow index, CFI. During the 1-minute coronary occlusion time, about 25% of the individuals in the study did not develop angina pectoris, and 20% did not develop intracoronary ECG signs of ischemia. Based on this information, it may be concluded that in general, if 100% - 82%, i.e. 82%, of the baseline flow is occluded, after 1 minute, approximately 20% of individuals would not develop signs of ischemia. This may be used as an upper limit, since 75% of the individuals did develop angina pectoris, and 80% of the individuals did develop ECG signs of ischemia. Hence the threshold time period $T_{ObsMax}$ during which an interventional device can safely obstruct $x$ % ($x \leq 82\%$) of a lumen of a collateral vessel in a retrograde CTO treatment procedure may be calculated in accordance with the equation:

$$T_{ObsMax}(\text{minutes}) = 0.82/x \qquad \text{Equation 1}$$

In the above equation, the value $x$ may be calculated as a ratio of the cross-sectional area of the interventional device used in the CTO treatment procedure to the cross sectional area of the collateral vessel. For $x > 0.82$, the value of $T_{ObsMax}$, may be restricted to 1 minute. In the event that additional collateral vessels feed tissue downstream of the occlusion, a revised value $x$ may be calculated as a ratio of the cross-sectional area of the interventional device used in the CTO treatment procedure to the total cross sectional area of all of the collateral vessels that feed tissue downstream of the occlusion.

The value of the threshold time period $T_{ObsMax}$ may alternatively be estimated using other techniques. For instance, in the example described above, it was assumed that the relationship between $T_{ObsMax}$ and $0.82/x$ in Equation 1 was linear. However, for smaller occlusions, $T_{ObsMax}$ may turn out to be longer, and consequently the relationship be non-linear. Thus, in another technique, the threshold time period $T_{ObsMax}$ during which an interventional device can safely obstruct $x$ % ($x \leq 82\%$) of a lumen of a collateral vessel in a retrograde CTO treatment procedure may be calculated in accordance with the equation:

$$T_{ObsMax}(\text{minutes}) = (0.82/x)^n \qquad \text{Equation 2}$$

and wherein $n \geq 1$. It is noted that other equations than these examples may alternatively be used to evaluate the threshold time period $T_{ObsMax}$. In other techniques, the value of the threshold time period may be evaluated based further on other factors, such as a type of the vessel, EHR data, ECG data, and so forth. For instance, elements of EHR data, and characteristics of ECG data, may be used to weight the values calculated above. In yet other techniques, other equations may alternatively be used to estimate the value of the threshold time period $T_{ObsMax}$.

The one or more processors 110 of the system 100 may obtain the value of the threshold time period $T_{ObsMax}$ using various techniques. For instance, in one technique, the threshold time period may be extracted from a lookup table based on the type of the collateral vessel. Other data relating to the collateral vessel may also be used to extract the threshold time period from the lookup table. For example, EHR data such as ECG data for the vessel may be used to extract the threshold time period from the lookup table by identifying in the lookup table, a threshold time period from a subject having similar EHR data. In another technique that is described in more detail below, the threshold time period $T_{ObsMax}$ may be predicted using a neural network.

As mentioned above, the obstruction time period $T_{obs}$ in the above example may be predicted using a neural network. In this example, the one or more processors 110 described above with reference to Fig. 3, are configured to analyze the second angiographic image data 190 to provide an estimate for an obstruction time period $T_{obs}$, by:

inputting the second angiographic image data 190 into a neural network 170 trained to estimate the obstruction time period $T_{obs}$; and
outputting the estimated obstruction time period $T_{obs}$ in response to the inputting; and
wherein the neural network is trained to estimate the obstruction time period based on the second angiographic image data 190.

This example is illustrated in Fig. 5, and wherein the estimated obstruction time period $T_{obs}$ is illustrated as being outputted in the central right-hand portion of the Figure.

In this example, the neural network 170 may be trained to estimate the obstruction time period $T_{obs}$ by including in the training data, a plurality of temporal sequences of angiographic images representing interventional devices in a lumen of a collateral vessel, and for each temporal sequence, corresponding ground truth data representing the obstruction time period $T_{obs}$, i.e. the total time during which the interventional device 200 has obstructed the lumen of the collateral vessel. The neural network 170 is then trained by inputting each temporal sequence of angiographic images, estimating the obstruction time period $T_{obs}$ using the neural network 170, and evaluating the loss function based further on a difference between the obstruction time period $T_{obs}$ estimated by the neural network, and the corresponding obstruction time period $T_{obs}$ from the ground truth data.

As mentioned above, the threshold time period $T_{ObsMax}$ in the above example may be predicted using a neural network. Two techniques are described below for this operation. In one technique, the threshold time period $T_{ObsMax}$ is predicted from angiographic image data. In this technique, the one or more processors 110 described with reference to Fig. 3 are configured to:

input the angiographic image data 120 and/or the second angiographic image data 190 into a neural network 170 trained to predict the threshold time period $T_{ObsMax}$ for the selected collateral vessel; and
output the threshold time period $T_{ObsMax}$ in response to the inputting; and
wherein the neural network 170 is trained to predict the threshold time period for the selected collateral vessel based on the inputted angiographic image data 120 and/or the second angiographic image data 190.

In another technique, the threshold time period $T_{ObsMax}$ is predicted from vessel parameters that are extracted from angiographic image data. In this technique, the one or more processors 110 described with reference to Fig. 3 are configured to:

extract, from the angiographic image data 120, one or more vessel parameters 220 for the selected collateral vessel;
input the extracted one or more vessel parameters 220 into a neural network 210 trained to predict the threshold time period $T_{ObsMax}$ for the selected collateral vessel; and
output the threshold time period $T_{ObsMax}$ for the selected collateral vessel in response to the inputting.

In this second technique, vessel parameters such as the type of the collateral vessel, a vessel supplied downstream of the collateral vessel, and a diameter of the collateral vessel, may be extracted from the angiographic image data 120. The vessel parameters 220 may be extracted using image processing techniques, or using neural networks that are trained to extract values for the one or more vessel parameters 220.

In both of these techniques, the neural network 170 may be trained to predict the threshold time period $T_{ObsMax}$, by:

receiving threshold time period training data, the threshold time period training data comprising, for each of a plurality of subjects, angiographic image data representing a collateral vessel used to perform a retrograde CTO treatment procedure, or extracted vessel parameters 220 for a collateral vessel used to perform a retrograde CTO treatment procedure;

receiving ground truth data corresponding to the threshold time period training data, the ground truth data comprising, for each of the plurality of subjects, an indication of a total time during which the interventional device can safely obstruct a lumen of the collateral vessel prior to an occurrence of a medical complication; and

for each of a plurality of the subjects:

inputting the threshold time period training data into the neural network 170, 210; and

adjusting parameters of the neural network based on a value of a loss function representing a difference between the threshold time period predicted by the neural network, and the corresponding total time from the ground truth data; and

repeating the inputting, and the adjusting, until a stopping criterion is met.

The training data that is used to train the neural network 170 in this example may be curated from historical procedures and in which the ground truth total time during which the interventional device can safely obstruct a lumen of the collateral vessel prior to an occurrence of a medical complication, i.e. $T_{ObsMax}$, has been determined by an expert. Alternatively, the value for $T_{ObsMax}$ used in the training data may be calculated from the temporal sequences of angiographic images used in the training data using Equation 1 above.

In a related example, electrocardiogram data for the occlusion may also be used to predict the threshold time period $T_{ObsMax}$. In this example, the neural network 170 is trained to predict the threshold time period $T_{ObsMax}$ based further on electrocardiogram data for the occlusion. In this example, the one or more processors 110 described with reference to Fig. 3, are configured to:

receive electrocardiogram data generated during the CTO treatment procedure;

input the electrocardiogram data into the neural network; and

output the threshold time period $T_{ObsMax}$ based further on the electrocardiogram data.

In this example, the neural network 170 is trained to predict the threshold time period $T_{ObsMax}$, by including in the threshold time period training data described above, electrocardiogram data for the corresponding subject. The use of the ECG data in accordance with this example helps to improve the accuracy of the neural network's predictions.

In another example, the neural network 170 is trained to predict the threshold time period $T_{ObsMax}$ based on a type of interventional device used in the CTO treatment procedure. In this example, the one or more processors 110 described with reference to Fig. 3, are configured to:

receive device data 230 comprising an identification of a type of interventional device used in the CTO treatment procedure;

input the device data 230 into the neural network; and

output the threshold time period $T_{ObsMax}$ based further on the type of the interventional device.

In this example, the neural network 170 is trained to predict the threshold time period $T_{ObsMax}$, by including in the threshold time period training data described above, a type of interventional device used in the CTO treatment procedure for the corresponding subject. The use of the type of the interventional device in accordance with this example helps to improve the accuracy of the neural network's predictions.

In another example, the neural network 170 is trained to predict the threshold time period $T_{ObsMax}$ based further on electronic health record data 180 relating to the occlusion 130; and wherein the one or more processors 110 are further configured to:

receive electronic health record data 180 relating to the occlusion 130; and

input the electronic health record data into the neural network; and

output the threshold time period $T_{ObsMax}$ based further on the electronic health record data 180.

The use of the type of the interventional device in accordance with this example helps to improve the accuracy of the neural network's predictions.

It is noted that in the examples described above, the system 100 may be provided with one or more additional items. For instance, the system 100 may also include one or more of: an angiographic imaging system 160 for providing the angiographic image data 120, 190, such as for example the projection X-ray imaging system illustrated in Fig. 3; an interventional device 200 for performing a retrograde CTO treatment procedure on the occlusion 130; a monitor 240 for displaying the data outputted by the one or more processors 110 such as graphical representations of the angiographic image data 120, 190, the identified collateral vessel(s) $150_{1..i}$ and their associated suitability score(s), and so forth; a patient bed 250; and a user input device (not illustrated in Fig. 3) such as a keyboard, a mouse, a touchscreen, and so forth, and which is configured to receive user input relating to operations performed by the one or more processors 110.

In another example, a computer-implemented method of selecting a collateral vessel for performing a retrograde chronic total occlusion, CTO, treatment procedure, is provided. The method comprises:

receiving S110 angiographic image data 120 representing an occlusion 130 in a target vessel 140;
analyzing S120 the angiographic image data 120 to identify one or more candidate collateral vessels $150_{1..i}$ for approaching the occlusion 130 in the target vessel 140 in a retrograde direction; and
assigning S130 a suitability score to each candidate collateral vessel $150_{1..i}$, the suitability score representing a suitability of the vessel for performing the retrograde CTO treatment procedure on the occlusion 130.

This method is illustrated in Fig. 4.

In another example, a computer program product comprising instructions which when executed by one or more processors 110, cause the one or more processors to carry out a method of selecting a collateral vessel for performing a retrograde chronic total occlusion, CTO, treatment procedure, is provided. The method comprises:

receiving S110 angiographic image data 120 representing an occlusion 130 in a target vessel 140;
analyzing S120 the angiographic image data 120 to identify one or more candidate collateral vessels $150_{1..i}$ for approaching the occlusion 130 in the target vessel 140 in a retrograde direction; and
assigning S130 a suitability score to each candidate collateral vessel $150_{1..i}$, the suitability score representing a suitability of the vessel for performing the retrograde CTO treatment procedure on the occlusion 130.

As mentioned above, a second aspect of the present disclosure relates to a system 300 for determining a threshold time period $T_{ObsMax}$. The threshold time period $T_{ObsMax}$ in this example corresponds to the threshold time period $T_{ObsMax}$ described above. However, in this second aspect, the threshold time period $T_{ObsMax}$ is provided by an independent system.

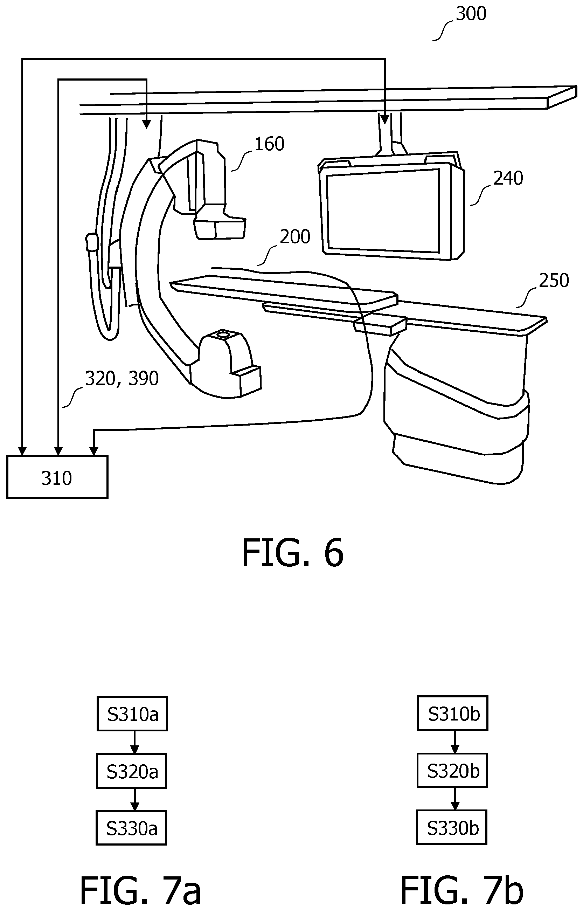

Fig. 6 is a schematic diagram illustrating an example of a system 300 for determining a threshold time period $T_{ObsMax}$, in accordance with some aspects of the present disclosure. Fig. 7a is a flowchart illustrating a first example of a computer-implemented method of determining a threshold time period $T_{ObsMax}$, in accordance with some aspects of the present disclosure. Fig. 7b is a flowchart illustrating a second example of a computer-implemented method of determining a threshold time period $T_{ObsMax}$, in accordance with some aspects of the present disclosure. The system 300 illustrated in Fig. 6 includes one or more processors 310. It is noted that operations described as being performed by the one or more processors 310, may also be performed as part of the methods illustrated in Fig. 7a and Fig. 7b. Moreover, it is noted that operations described as being performed as part of the method illustrated in Fig. 7a and Fig. 7b, may also be performed by the one or more processors illustrated in Fig. 6. It is also noted that the one or more processors 310 may be provided by the same one or more processors 110 illustrated in Fig. 3.

With reference to Fig. 6, the system 300 for determining a threshold time period $T_{ObsMax}$, the threshold time period representing a maximum total time during which an interventional device 200 can safely obstruct a lumen of a collateral vessel in a retrograde chronic total occlusion, CTO, treatment procedure, includes one or more processors 310 configured to:

receive S310a angiographic image data 320, the angiographic image data representing an occlusion 130 in a target vessel 140 and one or more collateral vessels $150_{1..i}$ for approaching the occlusion in the target vessel in a retrograde direction;
input S320a the angiographic image data 320 into a neural network 170 trained to predict the threshold time period $T_{ObsMax}$ for each of the one or more collateral vessels $150_{1..i}$; and output S330a the threshold time period $T_{ObsMax}$ for each of the one or more collateral vessels in response to the inputting; and
wherein the neural network 170 is trained to predict the threshold time period $T_{ObsMax}$ for each of the one or more collateral vessels based on the inputted angiographic image data.

The above operations are illustrated in the flowchart in Fig. 7a. The neural network 170 may be provided by a neural network such as the neural network 170 that was described above with reference to Fig. 5, and wherein the angiographic image data 120 inputted into the neural network 170 is instead provided by the angiographic image data 320, and the neural network 170 provides as output the threshold time period $T_{ObsMax}$. In this example, the other inputs to the neural network 170, i.e. the EHR data 180, and the second angiographic image data 190, may also be used but are not essential. In this example, the identified candidate collateral vessels $150_{1..i}$, their corresponding suitability score (s) illustrated in Fig. 5, and the Device type, are not outputted, and the obstruction time period $T_{Obs}$, may optionally be outputted, as described in the example below.

In this example, the neural network 170 illustrated in Fig. 5 may be trained to predict the threshold time period

$T_{ObsMax}$, by:

receiving threshold time period training data, the threshold time period training data comprising, for each of a plurality of subjects, angiographic image data representing a collateral vessel used to perform a retrograde CTO treatment procedure;

receiving ground truth data corresponding to the threshold time period training data, the ground truth data comprising, for each of the plurality of subjects, an indication of a total time during which the interventional device can safely obstruct a lumen of the collateral vessel prior to an occurrence of a medical complication; and

for each of a plurality of the subjects:

inputting the threshold time period training data into the neural network 170, 210; and

adjusting parameters of the neural network based on a value of a loss function representing a difference between the threshold time period predicted by the neural network, and the corresponding total time from the ground truth data; and

repeating the inputting, and the adjusting, until a stopping criterion is met.

In this example, the training data that is used to train the neural network 170 illustrated in Fig. 5 may be curated from historical procedures and in which the ground truth total time during which the interventional device can safely obstruct a lumen of the collateral vessel prior to an occurrence of a medical complication, i.e. $T_{ObsMax}$, has been determined by an expert.

In an alternative implementation, rather than predicting the threshold time period $T_{ObsMax}$ directly from the angiographic image data 320, vessel parameters are extracted from the angiographic image data 320, and the extracted vessel parameters 220 are inputted into a neural network 210 that is trained to predict the threshold time period $T_{ObsMax}$. The vessel parameters 220 may be extracted using image processing techniques, or using neural networks that are trained to extract values for the one or more vessel parameters 220.

In this implementation, the one or more processors 310 illustrated in Fig. 6 determine the threshold time period $T_{ObsMax}$ by performing the operations illustrated in Fig. 7b, rather than the operations illustrated in Fig. 7a. In this implementation, the system 300 for determining a threshold time period $T_{ObsMax}$, the threshold time period representing a maximum total time during which an interventional device 200 can safely obstruct a lumen of a collateral vessel in a retrograde chronic total occlusion, CTO, treatment procedure, includes one or more processors 310 configured to:

extract S310b, from the angiographic image data 320, one or more vessel parameters 220 for each of the one or more collateral vessels $150_{1..i}$,

input S320b the extracted one or more vessel parameters 220 into a neural network 210 trained to predict the threshold time period for each of the one or more collateral vessels; and

output S330b the threshold time period $T_{ObsMax}$ for each of the one or more collateral vessels in response to the inputting; and

wherein the neural network 210 is trained to predict the threshold time period for each of the one or more collateral vessels based on the inputted one or more vessel parameters 220.

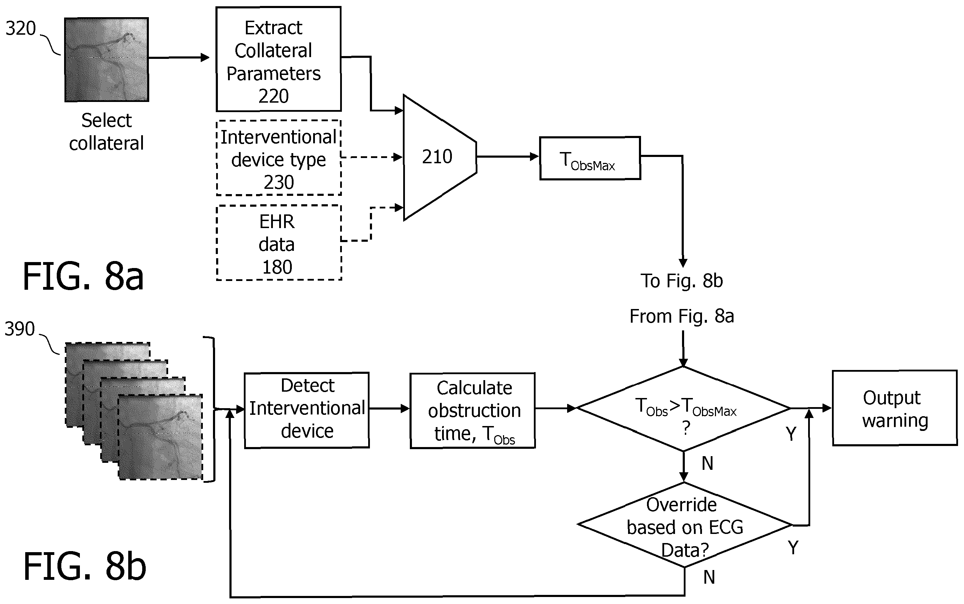

This implementation is also illustrated in Fig. 8a, which is a schematic diagram illustrating an example of a neural network 210 that is trained to predict a threshold time period, $T_{ObsMax}$, for a selected collateral vessel, based on extracted vessel parameters 220, in accordance with some aspects of the present disclosure.

In this example, the neural network 170 illustrated in Fig. 5 may be trained to predict the threshold time period $T_{ObsMax}$, by:

receiving threshold time period training data, the threshold time period training data comprising, for each of a plurality of subjects extracted vessel parameters 220 for a collateral vessel used to perform a retrograde CTO treatment procedure;

receiving ground truth data corresponding to the threshold time period training data, the ground truth data comprising, for each of the plurality of subjects, an indication of a total time during which the interventional device can safely obstruct a lumen of the collateral vessel prior to an occurrence of a medical complication; and

for each of a plurality of the subjects:

inputting the threshold time period training data into the neural network 170, 210; and

adjusting parameters of the neural network based on a value of a loss function representing a difference between the threshold time period predicted by the neural network, and the corresponding total time from the ground truth data; and

repeating the inputting, and the adjusting, until a stopping criterion is met.

In this example, the training data that is used to train the neural network 210 illustrated in Fig. 8a may include vessel parameters that have been curated from angiographic images for historical procedures and in which the ground truth total time during which the interventional device can safely obstruct a lumen of the collateral vessel prior to an occurrence of a medical complication, i.e. $T_{ObsMax}$, has been determined by an expert.

The threshold time period represents a maximum total time during which an interventional device can safely obstruct a lumen of a collateral vessel in a retrograde chronic total occlusion, CTO, treatment procedure. This information is useful to a physician in weighing-up the choice of collateral vessels for performing a retrograde CTO procedure. For instance, if the system indicates that a particular collateral vessel has a relatively shorter threshold time period as compared to other collateral vessels, the physician may decide to choose a different vessel that has a relatively longer threshold time period in order to safely complete the procedure within its expected duration. If none of the collateral vessels have a threshold time period that is long enough to accommodate the expected duration of the procedure, the physician may even decide to pursue an antegrade approach to the CTO.

As illustrated in Fig. 5, and in Fig. 8a, additional data may also be inputted into the neural networks 170, and 210 described above, and used to predict the threshold time period $T_{ObsMax}$. For instance, EHR data 180, or an indication of the interventional Device type 230, may also be inputted into the neural networks 170, and 210, whereupon the predicted threshold time period $T_{ObsMax}$ is generated based further on this data.

Returning to the system illustrated in Fig. 6. In one example, a warning is generated if the total time during which an interventional device 200 has obstructed a lumen of a collateral vessel exceeds a threshold time period. The warning may be provided in various ways, including visually, such as via a display device such as the monitor 240 illustrated in Fig. 3, or audially, and so forth. In another example, an indication of a remaining amount of time available to perform the procedure, is outputted. The warning, and similarly the indication of the remaining amount of time available to perform the procedure, alerts a physician to the elevated risk of complications during the retrograde CTO treatment procedure. In these examples, the one or more processors 310 are configured to:

receive input data representing an estimated obstruction time period $T_{Obs}$, the obstruction time period representing a total time during which the interventional device 200 has obstructed a lumen of a collateral vessel selected from the one or more collateral vessels $150_{1..i}$; and

output a warning in response to the estimated obstruction time period exceeding the threshold time period $T_{ObsMax}$; or output an indication of a remaining amount of time available to perform the procedure, the remaining amount of time being calculated based on a difference between the threshold time period $T_{ObsMax}$ and the obstruction time period $T_{obs}$.

In this example, the input data may be provided by various sources. For instance, in one example, the input data includes tracking generated by an interventional device tracking system. Tracking data may be generated by an interventional device tracking system configured to track a position of the interventional device during the retrograde CTO treatment procedure. The tracking data may be analyzed in order to evaluate the obstruction time period by tracking the position of a distal end of the interventional device with respect to a medical image representing the collateral vessel(s). Interventional device tracking systems such as electromagnetic tracking systems, optical fiber-based tracking systems may be used for this purpose. In another example, the input data includes user input data provided by a user. User input data may be provided in the form of an accumulated time from a clock that is started by a user when the interventional device enters the lumen. In another example, the input data is provided by a robotic system configured to control the interventional device. In another example, the input data includes a temporal sequence of images, and the temporal sequence of images is analyzed in order to estimate the obstruction time period $T_{Obs}$. In this example, the one or more processors 310 are configured to:

receive second angiographic image data 390 comprising a temporal sequence of images representing an interventional device 200 in a collateral vessel selected from the one or more collateral vessels $150_{1..i}$;

analyze the second angiographic image data 390 to provide an estimate for an obstruction time period $T_{Obs}$, the obstruction time period representing a total time during which the interventional device 200 has obstructed a lumen of the selected collateral vessel; and

output a warning in response to the estimated obstruction time period $T_{Obs}$ exceeding the threshold time period $T_{ObsMax}$; or

output an indication of a remaining amount of time available to perform the procedure, the remaining amount of time being calculated based on a difference between the threshold time period $T_{ObsMax}$ and the obstruction time period $T_{obs}$.

This example is illustrated in Fig. 8b, which is a schematic diagram illustrating an example of the analysis of second angiographic image data 390 in order to provide an estimate for an obstruction time period $T_{Obs}$, and the generation of a warning in response to the obstruction time period $T_{Obs}$ exceeding a threshold time period $T_{ObsMax}$, in accordance with some aspects of the present disclosure. In the example illustrated in Fig. 8b, the threshold time period $T_{ObsMax}$ is provided by the neural network 210. However, in another example, the threshold time period $T_{ObsMax}$ may alternatively be provided by the neural network 170 illustrated in Fig. 5, as described above.

In this example, the second angiographic image data 390 may be analyzed to provide an estimate for an obstruction time period $T_{obs}$ using various techniques. In one technique, image processing techniques such as feature detection may be used to detect the position of the interventional device in the temporal sequence of images in the second angiographic image data 390. In this technique, the obstruction time period $T_{obs}$ is estimated by detecting the position of the interventional device in relation to the selected collateral vessel in the images in the temporal sequence, and accumulating the total time during which the interventional device 200 is within the selected collateral vessel. In another technique, the obstruction time period $T_{obs}$ is estimated using a neural network. In this technique, the one or more processors 310 described above with reference to Fig. 6, are configured to analyze the second angiographic image data 390 to provide an estimate for an obstruction time period $T_{obs}$, by:

inputting the second angiographic image data 390 into a neural network 170 trained to estimate the obstruction time period $T_{obs}$; and

outputting the estimated obstruction time period $T_{obs}$ in response to the inputting; and

wherein the neural network is trained to estimate the obstruction time period based on the second angiographic image data 390.

This example is illustrated in Fig. 5, and wherein the estimated obstruction time period $T_{obs}$ is illustrated as being outputted in the right-hand portion of the Figure.

In this example, the neural network 170 may be trained to estimate the obstruction time period $T_{obs}$ by providing as the training data, a plurality of temporal sequences of angiographic images representing interventional devices in a lumen of a collateral vessel, and for each temporal sequence, corresponding ground truth data representing the obstruction time period $T_{obs}$, i.e. the total time during which the interventional device 200 has obstructed the lumen of the collateral vessel. The neural network is then trained by inputting each temporal sequence of angiographic images, estimating the obstruction time period $T_{obs}$ using the neural network 170, and evaluating the loss function based further on a difference between the obstruction time period $T_{obs}$ estimated by the neural network, and the corresponding obstruction time period $T_{obs}$ from the ground truth data.

It is noted that in the examples described above, the system 300 may be provided with one or more additional items. For instance, the system 300 may also include one or more of: an angiographic imaging system 160 for providing the angiographic image data 320, 390, such as for example the projection X-ray imaging system illustrated in Fig. 6; an interventional device 200 for performing a retrograde CTO treatment procedure on the occlusion 130; a monitor 240 for displaying the data outputted by the one or more processors 310 such as graphical representations of the angiographic image data 320, 390, the identified collateral vessel(s) $150_{1..i}$ and their associated suitability score(s), and so forth; a patient bed 250; and a user input device (not illustrated in Fig. 6) such as a keyboard, a mouse, a touchscreen, and so forth, and which is configured to receive user input relating to operations performed by the one or more processors 310.

In another example, a computer-implemented method of determining a threshold time period $T_{ObsMax}$, is provided. The threshold time period represents a maximum total time during which an interventional device 200 can safely obstruct a lumen of a collateral vessel in a retrograde chronic total occlusion, CTO, treatment procedure. The method comprises:

receiving S310a angiographic image data 320, the angiographic image data representing an occlusion 130 in a target vessel 140 and one or more collateral vessels $150_{1..i}$ for approaching the occlusion in the target vessel in a retrograde direction;

inputting S320a the angiographic image data 320 into a neural network 170 trained to predict the threshold time period $T_{ObsMax}$ for each of the one or more collateral vessels $150_{1..i}$; and outputting S330a the threshold time period $T_{ObsMax}$ for each of the one or more collateral vessels in response to the inputting; and

wherein the neural network 170 is trained to predict the threshold time period $T_{ObsMax}$ for each of the one or more collateral vessels based on the inputted angiographic image data;

or:

extracting S310b, from the angiographic image data 320, one or more vessel parameters 220 for each of the one or more collateral vessels $150_{1..i}$;

inputting S320b the extracted one or more vessel parameters 220 into a neural network 210 trained to predict the threshold time period for each of the one or more collateral vessels; and

outputting S330b the threshold time period $T_{ObsMax}$ for each of the one or more collateral vessels in response to the inputting; and

wherein the neural network 210 is trained to predict the threshold time period for each of the one or more collateral vessels based on the inputted one or more vessel parameters 220.

In another example, a computer program product is provided. The computer program product comprises instructions which when executed by one or more processors, cause the one or more processors to perform a method of determining a threshold time period $T_{ObsMax}$. The threshold time period represents a maximum total time during which an interventional device 200 can safely obstruct a lumen of a collateral vessel in a retrograde chronic total occlusion, CTO, treatment procedure. The method comprises:

receiving S310a angiographic image data 320, the angiographic image data representing an occlusion 130 in a target vessel 140 and one or more collateral vessels $150_{1..i}$ for approaching the occlusion in the target vessel in a retrograde direction;

inputting S320a the angiographic image data 320 into a neural network 170 trained to predict the threshold time period $T_{ObsMax}$ for each of the one or more collateral vessels $150_{1..i}$; and outputting S330a the threshold time period $T_{ObsMax}$ for each of the one or more collateral vessels in response to the inputting; and

wherein the neural network 170 is trained to predict the threshold time period $T_{ObsMax}$ for each of the one or more collateral vessels based on the inputted angiographic image data;

or:

extracting S310b, from the angiographic image data 320, one or more vessel parameters 220 for each of the one or more collateral vessels $150_{1..i}$;

inputting S320b the extracted one or more vessel parameters 220 into a neural network 210 trained to predict the threshold time period for each of the one or more collateral vessels; and

outputting S330b the threshold time period $T_{ObsMax}$ for each of the one or more collateral vessels in response to the inputting; and

wherein the neural network 210 is trained to predict the threshold time period for each of the one or more collateral vessels based on the inputted one or more vessel parameters 220.

The above examples are to be understood as illustrative of the present disclosure, and not restrictive. Further examples are also contemplated. For instance, the examples described in relation to a system, may also be provided as part of the corresponding computer-implemented method, or as part of the corresponding computer program product, or as part of the corresponding computer-readable storage medium. It is to be understood that a feature described in relation to any one example may be used alone, or in combination with other described features, and may be used in combination with one or more features of another of the examples, or a combination of other examples. Furthermore, equivalents and modifications not described above may also be employed without departing from the scope of the invention, which is defined in the accompanying claims. In the claims, the word "comprising" does not exclude other elements or operations, and the indefinite article "a" or "an" does not exclude a plurality. The mere fact that certain features are recited in mutually different dependent claims does not indicate that a combination of these features cannot be used to advantage. Any reference signs in the claims should not be construed as limiting their scope.

**Claims**

1. A system (100) for selecting a collateral vessel for performing a retrograde chronic total occlusion, CTO, treatment procedure, the system comprising one or more processors (110) configured to:

receive (S110) angiographic image data (120) representing an occlusion (130) in a target vessel (140);
analyze (S120) the angiographic image data (120) to identify one or more candidate collateral vessels ($150_{1..i}$) for approaching the occlusion (130) in the target vessel (140) in a retrograde direction; and
assign (S130) a suitability score to each candidate collateral vessel ($150_{1..i}$), the suitability score representing a suitability of the vessel for performing the retrograde CTO treatment procedure on the occlusion (130).

2. The system according to claim 1, wherein the one or more processors (110) are further configured to segment the angiographic image data (120) to identify the target vessel (140), and the occlusion (130), and the one or more candidate collateral vessels ($150_{1..i}$); and
wherein the one or more processors are configured to analyze the segmented angiographic image data to identify the one or more candidate collateral vessels ($150_{1..i}$).

3. The system according to claim 1 or claim 2, wherein the one or more processors (110) are configured to analyze the angiographic image data (120) by:

identifying one or more vessels having a connection to a distal position with respect to the occlusion (130), and for each identified vessel;
establishing a presence of a path between the connection and a proximal position with respect to the occlusion; and
assigning the vessel as a candidate collateral vessel ($150_{1..i}$) based on the presence of the path.

4. The system according to any one of claims 1 - 3, wherein the suitability score for each candidate collateral vessel ($150_{1..i}$) is evaluated based on one or more of the following factors:

a tortuosity of the candidate collateral vessel;
a tamponade risk value for the candidate collateral vessel;
a complexity of routing an interventional device along the candidate collateral vessel;
an ability to dilate the candidate collateral vessel;
electronic health record data relating to the occlusion;
blood flow data for the candidate collateral vessel.

5. The system according to any previous claim, wherein the angiographic image data (120) comprises cardiac angiographic image data, and wherein the candidate collateral vessels include: one or more septal collateral vessels ($150_1$, $150_2$) and/or one or more epicardial collateral vessels ($150_3$) and/or one or more bypass graft vessels ($150_4$).

6. The system according to any previous claim, wherein the one or more processors (110) are further configured to output an indication of a recommended orientation of a projection X-ray imaging system (160) for acquiring projection X-ray images of one or more of the identified candidate collateral vessels ($150_{1..i}$) during the CTO treatment procedure.

7. The system according to any previous claim, wherein the one or more processors (110) are further configured to analyze the angiographic image data (120) to identify one or more candidate collateral vessels ($150_{1..i}$) and/or to assign a suitability score to each candidate collateral vessel, by:

inputting the angiographic image data (120) into at least one neural network (170); and
outputting the identified one or more candidate collateral vessels ($150_{1..i}$) and/or the assigned one or more suitability scores, in response to the inputting; and
wherein the at least one neural network (170) is trained to identify the one or more candidate collateral vessels for approaching the occlusion in the target vessel in a retrograde direction and/or to assign a suitability score to each candidate collateral vessel, based on the inputted angiographic image data.

8. The system according to claim 7, wherein the at least one neural network (170) is trained to identify the one or more candidate collateral vessels and/or to assign a suitability score to each candidate collateral vessel, based further on electronic health record data (180) relating to the occlusion (130), and wherein the one or more processors (110) are further configured to:

receive electronic health record data (180) relating to the occlusion (130); and
input the electronic health record data into the at least one neural network (170);
such that the outputted one or more candidate collateral vessels and/or the assigned one or more suitability scores, is based further on the electronic health record data.

9. The system according to claim 7 or claim 8, wherein the at least one neural network (170) is trained to identify the one or more candidate collateral vessels and/or to assign a suitability score to each candidate collateral vessel, by:

receiving training data, the training data comprising, for each of a plurality of subjects: angiographic image data representing an occlusion in a target vessel;
receiving ground truth data corresponding to the training data, the ground truth data comprising, for each of the plurality of subjects: an identification of one or more collateral vessels for approaching the occlusion in the target vessel in a retrograde direction and/or a suitability score for each collateral vessel, the suitability score representing a suitability of the vessel for performing a retrograde CTO treatment procedure on the occlusion;

and

for each of a plurality of the subjects:

inputting the training data, into the at least one neural network; and
adjusting parameters of the at least one neural network based on a value of a loss function representing a difference between the one or more collateral vessels identified by the at least one neural network and/or the suitability score assigned to each collateral vessel by the at least one neural network, and the corresponding ground truth data; and
repeating the inputting, and the adjusting, until a stopping criterion is met.

10. The system according to any one of claims 7 - 9, wherein the at least one neural network (170) is further trained to predict a recommended type of an interventional device for use in the CTO treatment procedure, based on the inputted angiographic image data (120); and
wherein the one or more processors (110) are further configured to output the recommended type of the interventional device for use in the CTO treatment procedure, in response to the inputting.

11. The system according to any previous claim, wherein the one or more processors (110) are further configured to:

receive second angiographic image data (190) representing an interventional device (200) in the selected collateral vessel;
analyze the second angiographic image data (190) to provide an estimate for an obstruction time period ($T_{obs}$), the obstruction time period representing a total time during which the interventional device (200) has obstructed a lumen of the selected collateral vessel; and
output a warning in response to the estimated obstruction time period exceeding a threshold time period ($T_{ObsMax}$); or
output an indication of a remaining amount of time available to perform the procedure, the remaining amount of time being calculated based on a difference between the threshold time period ($T_{ObsMax}$) and the obstruction time period ($T_{obs}$).

12. The system according to claim 11, wherein the one or more processors (110) are further configured to:

input the angiographic image data (120) and/or the second angiographic image data (190) into a neural network (170) trained to predict the threshold time period ($T_{ObsMax}$) for the selected collateral vessel; and
output the threshold time period ($T_{ObsMax}$) in response to the inputting; and
wherein the neural network (170) is trained to predict the threshold time period for the selected collateral vessel based on the inputted angiographic image data (120) and/or the second angiographic image data (190);
or wherein the one or more processors (110) are further configured to:

extract, from the angiographic image data (120), one or more vessel parameters (220) for the selected collateral vessel;
input the extracted one or more vessel parameters (220) into a neural network (210) trained to predict the threshold time period ($T_{ObsMax}$) for the selected collateral vessel; and
output the threshold time period ($T_{ObsMax}$) for the selected collateral vessel in response to the inputting; and
wherein the neural network (210) is trained to predict the threshold time period ($T_{ObsMax}$) for the selected collateral vessel based on the inputted one or more vessel parameters (220).

13. The system according to claim 12, wherein the neural network (170) is trained to predict the threshold time period ($T_{ObsMax}$) based further on electrocardiogram data for the occlusion; and wherein the one or more processors (110) are further configured to:

receive electrocardiogram data generated during the CTO treatment procedure;
input the electrocardiogram data into the neural network; and
output the threshold time period ($T_{ObsMax}$) based further on the electrocardiogram data.

14. The system according to claim 13, wherein the neural network (170) is trained to predict the threshold time period ($T_{ObsMax}$) based further on a type of interventional device used in the CTO treatment procedure; and wherein the one or more processors (110) are further configured to:

receive device data (230) comprising an identification of a type of interventional device used in the CTO treatment procedure;

input the device data (230) into the neural network; and

output the threshold time period ($T_{ObsMax}$) based further on the type of the interventional device.

15. The system according to any one of claims 12 - 14, wherein the neural network (170) is trained to predict the threshold time period ($T_{ObsMax}$), by:

receiving threshold time period training data, the threshold time period training data comprising, for each of a plurality of subjects, angiographic image data representing a collateral vessel used to perform a retrograde CTO treatment procedure, or extracted vessel parameters (220) for a collateral vessel used to perform a retrograde CTO treatment procedure;

receiving ground truth data corresponding to the threshold time period training data, the ground truth data comprising, for each of the plurality of subjects, an indication of a total time during which the interventional device can safely obstruct a lumen of the collateral vessel prior to an occurrence of a medical complication; and

for each of a plurality of the subjects:

inputting the threshold time period training data into the neural network (170, 210); and

adjusting parameters of the neural network based on a value of a loss function representing a difference between the threshold time period predicted by the neural network, and the corresponding total time from the ground truth data; and

repeating the inputting, and the adjusting, until a stopping criterion is met.

**Patentansprüche**

1. System (100) zur Auswahl eines Kollateralgefäßes für die Durchführung eines retrograden Behandlungsverfahrens mit chronischer Totalokklusion, CTO, wobei das System einen oder mehrere Prozessoren (110) umfasst, die konfiguriert sind zum:

Empfangen (S110) von angiographischen Bilddaten (120), die eine Okklusion (130) in einem Zielgefäß (140) darstellen;

Analysieren (S 120) der angiographischen Bilddaten (120), um ein oder mehrere Kandidaten-Kollateralgefäße ($150_{1..i}$) zum Annähern an die Okklusion (130) im Zielgefäß (140) in einer retrograden Richtung zu identifizieren; und

Zuweisen (S130) eines Eignungswerts zu jedem Kandidaten-Kollateralgefäß ($150_{1..i}$), wobei der Eignungswert eine Eignung des Gefäßes zum Durchführen des retrograden CTO-Behandlungsverfahrens an der Okklusion (130) darstellt.

2. System nach Anspruch 1, wobei der eine oder die mehreren Prozessoren (110) weiter so konfiguriert sind, dass sie die angiographischen Bilddaten (120) segmentieren, um das Zielgefäß (140), die Okklusion (130) und das eine oder die mehreren Kandidaten-Kollateralgefäße ($150_{1..i}$) zu identifizieren; und

wobei der eine oder die mehreren Prozessoren so konfiguriert sind, dass sie die segmentierten angiographischen Bilddaten analysieren, um das eine oder die mehreren Kandidaten-Kollateralgefäße ($150_{1..i}$) zu identifizieren.

3. System nach Anspruch 1 oder Anspruch 2, wobei der eine oder die mehreren Prozessoren (110) so konfiguriert sind, dass sie die angiographischen Bilddaten (120) analysieren durch:

Identifizierung eines oder mehrerer Gefäße, die eine Verbindung zu einer distalen Position in Bezug auf die Okklusion (130) aufweisen und für jedes identifizierte Gefäß;

Feststellen eines Vorhandenseins eines Pfads zwischen der Verbindung und einer proximalen Position in Bezug auf die Okklusion; und

Zuordnen des Gefäßes als Kandidaten-Kollateralgefäß ($150_{1..i}$) basierend auf dem Vorhandensein des Pfades.

4. System nach einem der Ansprüche 1-3, wobei der Eignungswert für jedes Kandidaten-Kollateralgefäß ($150_{1..i}$) basierend auf einem oder mehreren der folgenden Faktoren beurteilt wird:

einer Verschlungenheit des Kandidaten-Kollateralgefäßes;

einen Tamponade-Risikowert für das Kandidaten-Kollateralgefäß;

einer Komplexität von Führung einer interventionellen Vorrichtung entlang des Kandidaten-Kollateralgefäßes;

einer Fähigkeit, das Kandidaten-Kollateralgefäß zu erweitern;

elektronischen Gesundheitsaktendaten in Bezug auf die Okklusion;

Blutflussdaten für des Kandidaten-Kollateralgefäß.

5. System nach einem vorstehenden Anspruch, wobei die angiographischen Bilddaten (120) kardiale angiographische Bilddaten umfassen, und wobei die Kandidaten-Kollateralgefäße Folgendes einschließen: ein oder mehrere septale Kollateralgefäße ($150_1$, $150_2$) und/oder ein oder mehrere epikardiale Kollateralgefäße ($150_3$) und/oder ein oder mehrere Bypass-Graft-Gefäße ($150_4$).

6. System nach einem vorstehenden Anspruch, wobei der eine oder die mehreren Prozessoren (110) weiter so konfiguriert sind, dass sie eine Angabe über eine empfohlene Ausrichtung eines Projektionsröntgenbildgebungssystems (160) zum Aufnehmen von Projektionsröntgenbildern eines oder mehrerer der identifizierten Kandidaten-Kollateralgefäße ($150_{1..i}$) während des CTO-Behandlungsverfahrens ausgeben.

7. System nach einem vorstehenden Anspruch, wobei der eine oder die mehreren Prozessoren (110) weiter so konfiguriert sind, dass sie die angiographischen Bilddaten (120) analysieren, um ein oder mehrere Kandidaten-Kollateralgefäße ($150_{1..i}$) zu identifizieren und/oder jedem Kandidaten-Kollateralgefäß einen Eignungswert zuzuweisen durch:

Eingeben der angiographischen Bilddaten (120) in mindestens ein neuronales Netzwerk (170); und

Ausgeben des identifizierten einen oder mehreren Kandidaten-Kollateralgefäße ($150_{1..i}$) und/oder des zugewiesenen einen oder mehreren Eignungswerte als Reaktion auf das Eingeben; und

wobei das mindestens eine neuronale Netzwerk (170) trainiert ist, um das eine oder die mehreren Kandidaten-Kollateralgefäße zum Annähern an die Okklusion im Zielgefäß in einer retrograden Richtung zu identifizieren und/oder jedem Kandidaten-Kollateralgefäß einen auf den eingegebenen angiographischen Bilddaten basierenden Eignungswert zuzuweisen.

8. System nach Anspruch 7, wobei das mindestens eine neuronale Netzwerk (170) trainiert ist, um das eine oder die mehreren Kandidaten-Kollateralgefäße zu identifizieren und/oder jedem Kandidaten-Kollateralgefäß einen weiter auf elektronischen Gesundheitsaktendaten (180) in Bezug auf die Okklusion (130) basierenden Eignungswert zuzuweisen, und wobei der eine oder die mehreren Prozessoren (110) weiter konfiguriert sind zum:

Empfangen elektronischer Gesundheitsaktendaten (180) in Bezug auf die Okklusion (130); und

Eingeben der elektronischen Gesundheitsaktendaten in das mindestens eine neuronale Netzwerk (170), sodass das ausgegebene eine oder mehreren Kandidaten-Kollateralgefäße und/oder der zugewiesene eine oder mehreren Eignungswerte weiter auf den elektronischen Gesundheitsaktendaten basieren.

9. System nach Anspruch 7 oder Anspruch 8, wobei das mindestens eine neuronales Netzwerk (170) trainiert ist, um das eine oder die mehreren Kandidaten-Kollateralgefäße zu identifizieren und/oder jedem Kandidaten-Kollateralgefäß einen Eignungswert zuzuweisen durch:

Empfangen von Trainingsdaten, wobei die Trainingsdaten für jedes einer Vielzahl von Subjekten Folgendes umfassen: angiographische Bilddaten, die eine Okklusion in einem Zielgefäß darstellen;

Empfangen von Grundwahrheitsdaten, die den Trainingsdaten entsprechen, wobei die Grundwahrheitsdaten für jedes der Vielzahl von Subjekten Folgendes umfassen: eine Identifizierung eines oder mehrerer Kollateralgefäße zur Annäherung an die Okklusion im Zielgefäß in einer retrograden Richtung und/oder einen Eignungswert für jedes Kollateralgefäß, wobei die Eignungswert eine Eignung des Gefäßes für die Durchführung eines retrograden CTO-Behandlungsverfahrens an der Okklusion darstellt; und

für jedes einer Vielzahl von Subjekten:

Eingeben der Trainingsdaten in das mindestens eine neuronale Netzwerk; und

Anpassen von Parametern des mindestens einen neuronalen Netzes basierend auf einem Wert einer Verlustfunktion, die eine Differenz zwischen dem einen oder den mehreren Kollateralgefäßen darstellt, die von dem mindestens einen neuronalen Netzwerk identifiziert wurden und/oder dem Eignungswert, die jedem Kollateralgefäß von den mindestens einen neuronalen Netzwerk zugewiesen wurde, und den entsprechenden Grundwahrheitsdaten; und

Wiederholen des Eingebens, und Anpassens, bis ein Abbruchkriterium erfüllt ist.

10. System nach einem der Ansprüche 7-9, wobei das mindestens eine neuronale Netzwerk (170) weiter trainiert ist, um basierend auf den eingegebenen angiographischen Bilddaten (120) einen empfohlenen Typ einer interventionellen Vorrichtung für die Verwendung im CTO-Behandlungsverfahren vorherzusagen; und wobei der eine oder die mehreren Prozessoren (110) weiter so konfiguriert sind, dass sie als Reaktion auf das Eingeben den empfohlenen Typ der interventionellen Vorrichtung für die Verwendung im CTO-Behandlungsverfahren ausgeben.

11. System nach einem der vorstehenden Ansprüche, wobei der eine oder die mehreren Prozessoren (110) weiter konfiguriert sind zum:

Empfangen von zweiten angiographischen Bilddaten (190), die eine interventionelles Vorrichtung (200) im ausgewählten Kollateralgefäß darstellen;

Analysieren der zweiten angiographischen Bilddaten (190), um eine Schätzung für eine Obstruktionszeitdauer ($T_{obs}$) bereitzustellen, wobei die Obstruktionszeitdauer eine Gesamtzeit darstellt, während der die interventionelle Vorrichtung (200) ein Lumen des ausgewählten Kollateralgefäßes obstruiert hat; und

Ausgaben einer Warnung als Reaktion darauf, dass die geschätzte Obstruktionszeitdauer eine Schwellenwertzeitdauer ($T_{ObsMax}$) überschreitet; oder

Ausgeben einer Angabe über die verbleibende Zeitmenge, die für die Durchführung der Prozedur zur Verfügung steht, wobei die verbleibende Zeit basierend auf einer Differenz zwischen der Schwellenwertzeitdauer ($T_{ObsMax}$) und der Obstruktionszeitdauer ($T_{obs}$) berechnet wird.

12. System nach Anspruch 11, wobei der eine oder die mehreren Prozessoren (110) weiter konfiguriert sind zum:

Eingeben der angiographischen Bilddaten (120) und/oder der zweiten angiographischen Bilddaten (190) in ein neuronales Netzwerk (170), das trainiert ist, um die Schwellenwertzeitdauer ($T_{ObsMax}$) für das ausgewählte Kollateralgefäß vorherzusagen; und

Ausgeben der Schwellenwertzeitdauer ($T_{ObsMax}$) als Reaktion auf das Eingeben; und wobei das neuronale Netzwerk (170) trainiert ist, um die Schwellenwertzeitdauer für das ausgewählte Kollateralgefäß basierend auf den eingegebenen angiographischen Bilddaten (120) und/oder den zweiten angiographischen Bilddaten (190) vorherzusagen;

oder wobei der eine oder die mehreren Prozessoren (110) weiter konfiguriert sind zum:

Extrahieren von einem oder mehreren Gefäßparametern (220) für das ausgewählte Kollateralgefäß aus den angiographischen Bilddaten (120);

Eingeben der einen oder mehreren extrahierten Gefäßparametern (220) in ein neuronales Netzwerk (210), das trainiert ist, um die Schwellenwertzeitdauer ($T_{ObsMax}$) für das ausgewählte Kollateralgefäß vorherzusagen; und

Ausgeben der Schwellenwertzeitdauer ($T_{ObsMax}$) für das ausgewählte Kollateralgefäß als Reaktion auf das Eingeben; und

wobei das neuronale Netzwerk (210) trainiert ist, um die Schwellenzeitdauer ($T_{ObsMax}$) für das ausgewählte Kollateralgefäß basierend auf dem einen oder den mehreren eingegebenen Gefäßparametern (220) vorherzusagen.

13. System nach Anspruch 12, wobei das neuronale Netzwerk (170) trainiert ist, um die Schwellenwertzeitdauer ($T_{ObsMax}$) weiter basierend auf Elektrokardiogrammdaten für die Okklusion vorherzusagen; und wobei der eine oder die mehreren Prozessoren (110) weiter konfiguriert sind zum:

Empfangen von während der CTO-Behandlungsprozedur generierten Elektrokardiogrammdaten;

Eingeben der Elektrokardiogrammdaten in das neuronale Netzwerk; und

Ausgeben der Schwellenwertdauer ($T_{ObsMax}$) weiter basierend auf den Elektrokardiogrammdaten.

14. System nach Anspruch 13, wobei das neuronale Netzwerk (170) trainiert ist, um die Schwellenwertzeitdauer ($T_{ObsMax}$) basierend auf einem Typ in der CTO-Behandlungsprozedur verwendeten von interventioneller Vorrichtung vorherzusagen; und wobei der eine oder die mehreren Prozessoren (110) weiter konfiguriert sind zum:

Empfangen von Vorrichtungsdaten (230), die eine Identifizierung eines Typs der in der CTO-Behandlungsprozedur verwendeten interventionellen Vorrichtung umfassen;

Eingeben der Vorrichtungsdaten (230) in das neuronale Netzwerk; und

Ausgeben der Schwellenwertzeitdauer ($T_{ObsMax}$) weiter basierend auf dem Typ der interventionellen Vorrichtung.

15. System nach einem der Ansprüche 12 - 14, wobei das neuronale Netzwerk (170) trainiert ist, um die Schwellenwertzeitdauer ($T_{ObsMax}$) vorherzusagen durch:

Empfangen von Trainingsdaten für die Schwellenwertzeitdauer, wobei die Trainingsdaten für die Schwellenwertzeitdauer, für jedes einer Vielzahl von Subjekten, angiographische Bilddaten umfassen, die ein Kollateralgefäß darstellen, das zur Durchführung einer retrograden CTO-Behandlungsprozedur verwendet wird, oder extrahierte Gefäßparameter (220) für ein Kollateralgefäß, das zur Durchführung einer retrograden CTO-Behandlungsprozedur verwendet wird;

Empfangen von Grundwahrheitsdaten, die den Trainingsdaten für den Schwellenwertzeitraum entsprechen, wobei die Grundwahrheitsdaten, für jedes der Vielzahl von Subjekten, eine Angabe einer Gesamtzeitdauer enthalten, während der die interventionelle Vorrichtung ein Lumen des Kollateralgefäßes sicher obstruieren kann, bevor eine medizinische Komplikation auftritt; und

für jedes einer Vielzahl von Subjekten:

Eingeben der Trainingsdaten für die Schwellenwertzeitdauer in das neuronale Netzwerk (170, 210); und

Anpassen von Parametern des neuronalen Netzwerks basierend auf einem Wert einer Verlustfunktion, die eine Differenz zwischen der vom neuronalen Netzwerk vorhergesagten Schwellenwertzeitdauer und der entsprechenden Gesamtzeit aus den Referenzdaten darstellt; und

Wiederholen des Eingebens, und Anpassens, bis ein Abbruchkriterium erfüllt ist.

## Revendications

1. Système (100) de sélection d'un vaisseau collatéral pour effectuer une procédure de traitement d'occlusion totale chronique (CTO) rétrograde, le système comprenant un ou plusieurs processeurs (110) configurés pour :

recevoir (S110) des données d'image angiographique (120) représentant une occlusion (130) dans un vaisseau cible (140) ;

analyser (S120) les données d'image angiographique (120) pour identifier un ou plusieurs vaisseaux collatéraux candidats ($150_{1..i}$) pour aborder l'occlusion (130) dans le vaisseau cible (140) en direction rétrograde ; et

attribuer (S130) un score d'adéquation à chaque vaisseau collatéral candidat ($150_{1..i}$), le score d'adéquation représentant l'adéquation du vaisseau pour effectuer la procédure de traitement CTO rétrograde sur l'occlusion (130).

2. Système selon la revendication 1, dans lequel les un ou plusieurs processeurs (110) sont en outre configurés pour segmenter les données d'image angiographique (120) afin d'identifier le vaisseau cible (140), l'occlusion (130) et les un ou plusieurs vaisseaux collatéraux candidats ($150_{1..i}$) ; et

dans lequel les un ou plusieurs processeurs sont configurés pour analyser les données d'image angiographique segmentées afin d'identifier les un ou plusieurs vaisseaux collatéraux candidats ($150_{1..i}$).

3. Système selon la revendication 1 ou la revendication 2, dans lequel les un ou plusieurs processeurs (110) sont configurés pour analyser les données d'image angiographique (120) par :

l'identification d'un ou plusieurs vaisseaux présentant une connexion à une position distale par rapport à l'occlusion (130), et pour chaque vaisseau identifié ;

l'établissement de la présence d'un chemin entre la connexion et une position proximale par rapport à l'occlusion ; et

l'attribution du vaisseau comme vaisseau collatéral candidat ($150_{1..i}$) en fonction de la présence du chemin.

4. Système selon l'une quelconque des revendications 1-3, dans lequel le score d'adéquation de chaque vaisseau collatéral candidat ($150_{1..i}$) est évalué sur la base d'un ou plusieurs des facteurs suivants :

EP 4 719 198 B1

une tortuosité du vaisseau collatéral candidat ;
une valeur de risque de tamponnade pour le vaisseau collatéral candidat ;
une complexité du routage d'un dispositif interventionnel le long du vaisseau collatéral candidat ;
une capacité à dilater le vaisseau collatéral candidat ;
des données du dossier médical électronique relatives à l'occlusion ;
des données relatives au flux sanguin du vaisseau collatéral candidat.

5. Système selon une quelconque revendication précédente, dans lequel les données d'image angiographique (120) comprennent des données d'image angiographique cardiaque, et dans lequel les vaisseaux collatéraux candidats incluent : un ou plusieurs vaisseaux collatéraux septaux ($150_1$, $150_2$) et/ou un ou plusieurs vaisseaux collatéraux épicardiques ($150_3$) et/ou un ou plusieurs vaisseaux greffés de pontage ($150_4$).

6. Système selon une quelconque revendication précédente, dans lequel les un ou plusieurs processeurs (110) sont en outre configurés pour fournir une indication d'une orientation recommandée d'un système d'imagerie par rayons X de projection (160) pour l'acquisition d'images par rayons X de projection d'un ou plusieurs des vaisseaux collatéraux candidats identifiés ($150_{1..i}$) pendant la procédure de traitement CTO.

7. Système selon une quelconque revendication précédente, dans lequel les un ou plusieurs processeurs (110) sont en outre configurés pour analyser les données d'image angiographique (120) afin d'identifier un ou plusieurs vaisseaux collatéraux candidats ($150_{1..i}$) et/ou pour attribuer un score d'adéquation à chaque vaisseau collatéral candidat par :

entrée des données d'image angiographique (120) dans au moins un réseau neuronal (170) ; et
sortie des un ou plusieurs vaisseaux collatéraux candidats identifiés ($150_{1..i}$) et/ou des un ou plusieurs scores d'adéquation attribués, en réponse à l'entrée ; et
dans lequel le au moins un réseau neuronal (170) est entraîné à identifier les un ou plusieurs vaisseaux collatéraux candidats pour aborder l'occlusion du vaisseau cible dans une direction rétrograde et/ou pour attribuer un score d'adéquation à chaque vaisseau collatéral candidat, en fonction des données d'image angiographique entrées.

8. Système selon la revendication 7, dans lequel le au moins un réseau neuronal (170) est entraîné à identifier les un ou plusieurs vaisseaux collatéraux candidats et/ou à attribuer un score d'adéquation à chaque vaisseau collatéral candidat, sur la base en outre des données du dossier médical électronique (180) relatives à l'occlusion (130), et dans lequel les un ou plusieurs processeurs (110) sont en outre configurés pour :

recevoir des données du dossier médical électronique (180) relatives à l'occlusion (130) ; et
entrer les données du dossier médical électronique dans le au moins un réseau neuronal (170) ; de sorte que les un ou plusieurs vaisseaux collatéraux candidats sortis et/ou les un ou plusieurs scores d'adéquation attribués sont basés sur les données du dossier médical électronique.

9. Système selon la revendication 7 ou 8, dans lequel le au moins un réseau neuronal (170) est entraîné à identifier les un ou plusieurs vaisseaux collatéraux candidats et/ou à attribuer un score d'adéquation à chaque vaisseau collatéral candidat par :

réception de données d'entraînement, les données d'entraînement comprenant, pour chacun d'une pluralité de sujets : des données d'image angiographique représentant une occlusion dans un vaisseau cible ;
réception de données de réalité de terrain correspondant aux données d'entraînement, les données de réalité de terrain comprenant, pour chacun de la pluralité de sujets : une identification d'un ou plusieurs vaisseaux collatéraux permettant d'aborder l'occlusion du vaisseau cible dans une direction rétrograde et/ou un score d'adéquation pour chaque vaisseau collatéral, le score d'adéquation représentant l'adéquation du vaisseau à la réalisation d'une procédure de traitement CTO rétrograde sur l'occlusion ; et
pour chacun d'une pluralité des sujets :

l'entrée des données d'entraînement dans le au moins un réseau neuronal ; et
l'ajustement des paramètres de l'au moins un réseau neuronal en fonction de la valeur d'une fonction de perte représentant une différence entre les un ou plusieurs vaisseaux collatéraux identifiés par le au moins un réseau neuronal et/ou le score d'adéquation attribué à chaque vaisseau collatéral par le au moins un réseau neuronal, et les données de réalité de terrain correspondantes ; et

27

la répétition de l'entrée et de l'ajustement jusqu'à ce qu'un critère d'arrêt soit atteint.

10. Système selon l'une quelconque des revendications 7-9, dans lequel le au moins un réseau neuronal (170) est en outre entraîné à prédire un type recommandé de dispositif interventionnel à utiliser dans la procédure de traitement CTO, sur la base des données d'image angiographique entrées (120) ; et
dans lequel les un ou plusieurs processeurs (110) sont en outre configurés pour fournir le type recommandé de dispositif interventionnel à utiliser dans la procédure de traitement CTO, en réponse à l'entrée.

11. Système selon une quelconque revendication précédente, dans lequel les un ou plusieurs processeurs (110) sont en outre configurés pour :

recevoir des secondes données d'image angiographique (190) représentant un dispositif interventionnel (200) dans le vaisseau collatéral sélectionné ;
analyser les secondes données d'image angiographique (190) pour fournir une estimation de la durée d'obstruction ($T_{obs}$), la durée d'obstruction représentant le temps total pendant lequel le dispositif interventionnel (200) a obstrué la lumière du vaisseau collatéral sélectionné ; et
émettre un avertissement lorsque la durée d'obstruction estimée dépasse une durée seuil ($T_{ObsMax}$) ; ou
fournir une indication du temps restant disponible pour effectuer la procédure, le temps restant étant calculé sur la base d'une différence entre la durée seuil ($T_{ObsMax}$) et la durée d'obstruction ($T_{obs}$).

12. Système selon la revendication 11, dans lequel les un ou plusieurs processeurs (110) sont en outre configurés pour :

entrer les données d'image angiographique (120) et/ou les secondes données d'image angiographique (190) dans un réseau neuronal (170) entraîné à prédire la durée seuil ($T_{ObsMax}$) pour le vaisseau collatéral sélectionné ; et
fournir la durée seuil ($T_{ObsMax}$) en réponse à l'entrée ; et dans lequel le réseau neuronal (170) est entraîné à prédire la durée seuil pour le vaisseau collatéral sélectionné sur la base des données d'image angiographique entrées (120) et/ou les secondes données d'image angiographique (190) ;
ou dans lequel les un ou plusieurs processeurs (110) sont en outre configurés pour :

extraire, à partir des données d'image angiographique (120), un ou plusieurs paramètres de vaisseau (220) pour le vaisseau collatéral sélectionné ;
entrer les un ou plusieurs paramètres extraits du vaisseau (220) dans un réseau neuronal (210) entraîné à prédire la durée seuil ($T_{ObsMax}$) pour le vaisseau collatéral sélectionné ; et
fournir la durée seuil ($T_{ObsMax}$) pour le vaisseau collatéral sélectionné en réponse à l'entrée ; et
dans lequel le réseau neuronal (210) est entraîné à prédire la durée seuil ($T_{ObsMax}$) pour le vaisseau collatéral sélectionné en fonction des un ou plusieurs paramètres de vaisseau entrés (220).

13. Système selon la revendication 12, dans lequel le réseau neuronal (170) est entraîné à prédire la durée seuil ($T_{ObsMax}$) sur la base des données d'électrocardiogramme de l'occlusion ; et dans lequel les un ou plusieurs processeurs (110) sont en outre configurés pour :

recevoir les données d'électrocardiogramme générées pendant la procédure de traitement CTO ;
entrer les données d'électrocardiogramme dans le réseau neuronal ; et
fournir la durée seuil ($T_{ObsMax}$) sur la base en outre des données d'électrocardiogramme.

14. Système selon la revendication 13, dans lequel le réseau neuronal (170) est entraîné à prédire la durée seuil ($T_{ObsMax}$) sur la base en outre du type de dispositif interventionnel utilisé dans la procédure de traitement CTO ; et dans lequel les un ou plusieurs processeurs (110) sont en outre configurés pour :

recevoir des données de dispositif (230) comprenant une identification d'un type de dispositif interventionnel utilisé dans la procédure de traitement CTO ;
entrer les données du dispositif (230) dans le réseau neuronal ; et
fournir la durée seuil ($T_{ObsMax}$) sur la base en outre du type de dispositif d'intervention.

15. Système selon l'une quelconque des revendications 12-14, dans lequel le réseau neuronal (170) est entraîné à prédire la durée seuil ($T_{ObsMax}$) par :

réception des données d'entraînement de durée seuil, les données d'entraînement de durée seuil comprenant, pour chacun d'une pluralité de sujets, des données d'image angiographique représentant un vaisseau collatéral utilisé pour effectuer une procédure de traitement CTO rétrograde, ou des paramètres de vaisseau extraits (220) pour un vaisseau collatéral utilisé pour effectuer une procédure de traitement CTO rétrograde ;

réception de données de réalité de terrain correspondant aux données d'entraînement de durée seuil, les données de réalité de terrain comprenant, pour chacun de la pluralité de sujets, une indication du temps total pendant lequel le dispositif interventionnel peut obstruer en toute sécurité la lumière du vaisseau collatéral avant la survenue d'une complication médicale ; et

pour chacun d'une pluralité de sujets :

entrée des données d'entraînement de durée seuil dans le réseau neuronal (170, 210) ; et

ajustement des paramètres du réseau neuronal en fonction de la valeur d'une fonction de perte représentant la différence entre la durée seuil prédite par le réseau neuronal et le temps total correspondant issu des données de réalité de terrain ; et

répéter l'entrée et l'ajustement jusqu'à ce qu'un critère d'arrêt soit atteint.

FIG. 1

FIG. 2

# FIG. 3

# FIG. 4

FIG. 5

FIG. 6

FIG. 7a

FIG. 7b

320

Select
collateral

Extract
Collateral
Parameters
220

Interventional
device type
230

EHR
data
180

210

$T_{ObsMax}$

## FIG. 8a

To Fig. 8b

From Fig. 8a

390

Detect
Interventional
device

Calculate
obstruction
time, $T_{Obs}$

$T_{Obs} > T_{ObsMax}$
?

Y

N

Override
based on ECG
Data?

Y

N

Output
warning

## FIG. 8b

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **MEGALY, M. et al.** Retrograde Approach to Chronic Total Occlusion Percutaneous Coronary Intervention. *Circ. Cardiovasc. Interv.*, 2020, vol. 13, e008900
- **CHING-CHANG HUANG**. Collateral Channel Size and Tortuosity Predict Retrograde Percutaneous Coronary Intervention Success for Chronic Total Occlusion. *Circulation: Cardiovascular Interventions 2018*, 01 January 2018, vol. 11 (1)

- **WUSTMANN, K et al.** Is There Functional Collateral Flow During Vascular Occlusion in Angiographically Normal Coronary Arteries?. *Circulation*, 2003, vol. 107, 2213-2220